# EUROPÄISCHE PATENTSCHRIFT

(11) **EP 3 706 636 B1**
(45) Veröffentlichungstag und Bekanntmachung des Hinweises auf die Patenterteilung: **01.05.2024**
(21) Anmeldenummer: 18800586.2
(22) Anmeldetag: 07.11.2018
(51) Int. Cl.: A61B 8/08, A61B 8/14, G16H 30/20, G16H 50/20

(54) **ULTRASCHALL-BILDERZEUGUNGSSYSTEM**
ULTRASOUND IMAGE GENERATING SYSTEM
SYSTÈME D'IMAGERIE PAR ULTRASONS

(30) Priorität: 08.11.2017 DE 102017126158
(43) Veröffentlichungstag der Anmeldung: 16.09.2020
(73) Patentinhaber: Fraunhofer-Gesellschaft zur Förderung der angewandten Forschung e.V., 80686 München (DE)
(72) Erfinder: GÜNTHER, Matthias, 28359 Bremen (DE)
(74) Vertreter: Eisenführ Speiser
(86) Internationale Anmeldenummer: PCT/EP2018/080495
(87) Internationale Veröffentlichungsnummer: WO 2019/092033

(56) Entgegenhaltungen:
- CN-A- 106 373 109
- US-A1- 2016 113 630
- SANKETH VEDULA ET AL: "Towards CT-quality Ultrasound Imaging using Deep Learning", ARXIV.ORG, CORNELL UNIVERSITY LIBRARY, 201 OLIN LIBRARY CORNELL UNIVERSITY ITHACA, NY 14853, 17. Oktober 2017 (2017-10-17), XP080825022,
- PREISWERK FRANK ET AL: "Synthesizing Dynamic MRI Using Long-Term Recurrent Convolutional Networks", 15. September 2018 (2018-09-15), INTERNATIONAL CONFERENCE ON SIMULATION, MODELING, AND PROGRAMMING FOR AUTONOMOUS ROBOTS,SIMPAR 2010; [LECTURE NOTES IN COMPUTER SCIENCE; LECT.NOTES COMPUTER], SPRINGER, BERLIN, HEIDELBERG, PAGE(S) 89 - 97, XP047485712, ISBN: 978-3-642-17318-9 [gefunden am 2018-09-15] das ganze Dokument

## Beschreibung

Die Erfindung bezieht sich auf ein Ultraschall-Bilderzeugungssystem, ein Ultraschall-Bilderzeugungsverfahren und ein Computerprogramm zum Erzeugen eines Bildes eines Objekts unter Verwendung von Ultraschallrohdaten.

Die Ultraschall-Bildgebung wird in der medizinischen Diagnostik sehr häufig eingesetzt.

Sie ist verhältnismäßig kostengünstig und flexibel in der Anwendung. Sie ermöglicht den mobilen Einsatz und bietet Echtzeitbildgebung mit beispielsweise bis zu 50 Ultraschallbildem pro Sekunde. Ein Nachteil der Ultraschall-Bildgebung ist allerdings die vergleichsweise geringe Bildqualität.

Es ist eine Aufgabe der vorliegenden Erfindung, ein Ultraschall-Bildgebungssystem, ein Ultraschall-Bildgebungsverfahren und ein Computerprogramm zum Erzeugen eines Bildes eines Objekts bereitzustellen, die eine verbesserte Bildqualität ermöglichen.

In Sanketh Vedula et al.,"Towards CT-Quality Ultrasound Imaging Using Deep Learning", arXiv:1710.06304, ist ein gefaltetes neuronales Netzwerk beschrieben, um Ultraschallbilddaten in CT-ähnliche Bilder zu konvertieren. Der nach dem beanspruchten Prioritätsdatum der vorliegenden Anmeldung veröffentlichte Artikel von Frank Preiswerk, Cheng-Chieh Cheng, die Luo und Bruno Madore, "Synthesizing Dynamic MRI Using Long-Term Recurrent Convolutional Networks", in Machine Learning in Medical Imaging (MLMI) 2018, Lecture Notes in Computer Science Vol. 11046, offenbart das Konvertieren von Ultraschallrohdaten in MRI-ähnliche Bilder durch eine mittels Maschinenlernens trainierte Einheit.

Die oben genannte Aufgabe wird durch ein Ultraschall-Bilderzeugungssystem zum Erzeugen eines Bildes eines Objekts gelöst, wobei das Ultraschall-Bilderzeugungssystem aufweist:
- eine Ultraschalldatenbereitstellungseinheit zum Bereitstellen von Ultraschallrohdaten des Objekts, die mit einer Ultraschallbildungsmodalität aufgenommen worden sind,
- eine Trainierte-Einheit-Bereitstellungseinheit zum Bereitstellen einer mittels Maschinenlernens trainierten Einheit, die angepasst ist, auf Basis von Ultraschallrohdaten eines Objekts ein Ultraschall-Bild des Objekts zu erzeugen, das nicht der Ultraschallbildgebungsmodalität entspricht, die zur Aufnahme der Ultraschallrohdaten verwendet worden ist, und
- eine Bilderzeugungseinheit zum Erzeugen des Bildes des Objekts unter Verwendung der bereitgestellten trainierten Einheit auf Basis der bereitgestellten Ultraschallrohdaten des Objekts.

Die Erfindung ist in den Ansprüchen definiert.

Ultraschallrohdaten beinhalten sehr viele Informationen über ein Objekt, die von bekannten Ultraschall-Rekonstruktionsverfahren nur zu einem sehr kleinen Bruchteil verwendet werden. Diese Nichtberücksichtigung von einem großen Teil der Informationen, die in den Ultraschallrohdaten enthalten sind, führt u.a. zu der relativ schlechten Bildqualität und Kontrastarmut bekannter Ultraschallbilder. Es ist allerdings nicht bekannt, wie diese zusätzlichen Informationen genutzt werden können, um die Bildqualität zu verbessern.

Es wurde nun erkannt, dass diese bisher nicht berücksichtigten Informationen, die in den Ultraschallrohdaten enthalten sind, mithilfe einer mittels Maschinenlernens trainierten Einheit wie eines neuronalen Netzes extrahiert werden können, die nicht zur Rekonstruktion von Ultraschallbildern verwendet wird, die der Ultraschallbildgebungsmodalität entsprechen, die zur Erzeugung der Ultraschallrohdaten verwendet wurde. Das heißt, mit Hilfe der trainierten Einheit können auf Basis der Ultraschallrohdaten Ultraschallbilder erzeugt werden, die nicht den Ultraschallbildern entsprechen, die "normalerweise" mittels der Ultraschallbildgebungsmodalität erzeugt werden können. Stattdessen weisen die mittels der trainierten Einheit erzeugten Ultraschallbilder Charakteristika auf, die einer anderen Bildgebungsmodalität entsprechen, wobei die in den Ultraschallrohdaten enthaltenen Informationen besser ausgenutzt werden können. Beispielsweise kann das Ultraschall-Bilderzeugungssystem durch Verwendung der trainierten Einheit ein Ultraschallbild rekonstruieren, das die Charakteristik eines Computertomographie-Bildes (CT-Bild), eines Magnetresonanz-Bildes (MR-Bild), eines Positronen-Emissions-Tomographie-Bildes (PET-Bild), eines Einzelphotonen-Emissions-Computertomographie-Bildes (SPECT-Bild) oder eines Magnetpartikel-Bildes (MPI-Bild) aufweist. Auch die Rekonstruktion eines anderen tomographischen Ultraschallbildes, das nicht der Ultraschallbildgebungsmodalität entspricht, ist möglich. Das mittels der trainierten Einheit erzeugte Ultraschallbild unterscheidet sich demnach von einem "normalen" Ultraschallbild, das normalerweise von der Ultraschallbildgebungsmodalität erzeugt wird, mit dem die Ultraschallrohdaten aufgenommen worden sind, dadurch, dass das mit der trainierten Einheit erzeugte Ultraschallbild Charakteristika einer anderen Bildgebungsmodalität aufweist. Durch die bessere Ausnutzung der in den Ultraschallrohdaten enthaltenen Informationen über das Objekt können die Bildqualität und der Informationsgehalt ("Kontrast") verbessert werden.

Grundsätzlich erzeugt jede Bildgebungsmodalität ein für die entsprechende Bildgebungsmodalität charakteristisches Bild. Das heißt, es ist bekannt, dass es für jede Bildgebungsmodalität spezifische Bild-Charakteristika gibt, die es ermöglichen, ein Bild der entsprechenden Bildgebungsmodalität zuzuordnen. Ein CT-Bild hat bspw. CT-Bild-Charakteristika, so dass dem Fachmann klar ist, dass das CT-Bild einer CT-Bildgebungsmodalität entspricht. Ein Ultraschallbild, das nicht der Ultraschallbildgebungsmodalität entspricht, mit dem die Ultraschallrohdaten aufgenommen worden sind, ist ein Ultraschallbild, das diese Bild-Charakteristika aufweist, die einer anderen Bildgebungsmodalität entsprechen.

Bild-Charakteristika in einem Bild eines Objekts, die einer Bildgebungsmodalität entsprechen, die nicht die zur Aufnahme der Ultraschallrohdaten verwendete Ultraschallbildgebungsmodalität ist, sind bevorzugt Objektcharakteristika des in dem Bild gezeigten Objekts, die in einem Ultraschallbild des Objekts, das mit der Ultraschallbildgebungsmodalität erzeugt worden wäre, mit der die Ultraschallrohdaten aufgenommen worden sind, nicht sichtbar wären. So können in dem mittels der bereitgestellten trainierten Einheit erzeugten Ultraschallbild beispielsweise Eigenschaften des Objekts sichtbar sein, die auch in einem MR-Bild oder einem Bild einer anderen Bildgebungsmodalität sichtbar wären, aber nicht in einem Ultraschallbild der Ultraschallbildgebungsmodalität, die zur Aufnahme der Ultraschallrohdaten verwendet worden ist.

Die von der Trainierte-Einheit-Bereitstellungseinheit bereitgestellte trainierte Einheit verwendet als Eingang Ultraschallrohdaten und nicht Ultraschallbilder. Bei einer bekannten Standardrekonstruktion eines Ultraschallbildes geht ein Großteil der in den Ultraschallrohdaten enthaltenen Objektinformationen verloren. Dieser Informationsverlust kann unter anderem dadurch vermieden werden, dass die bereitgestellte trainierte Einheit als Eingang die Ultraschallrohdaten verwendet und nicht bereits rekonstruierte Ultraschallbilder.

Das mittels der bereitgestellten trainierten Einheit rekonstruierte Ultraschallbild ist immer noch ein Ultraschallbild und weist im Allgemeinen typische Ultraschallbildcharakteristika auf wie Speckle-Muster oder einen Tiefenabfall. Dieses rekonstruierte Ultraschallbild zeigt aber auch Charakteristika des Objekts, die in einem "normalen" Ultraschallbild der zur Aufnahme der Ultraschallrohdaten verwendeten Ultraschallbildgebungsmodalität nicht sichtbar wären, aber beispielsweise in einem MR-Bild oder einem Bild einer anderen Bildgebungsmodalität. Von welcher Bildgebungsmodalität, die nicht die Ultraschallbildgebungsmodalität ist, die zur Aufnahme der Ultraschallrohdaten verwendet worden ist, die Charakteristika in dem Ultraschallbild sichtbar sind, hängt von der Bildgebungsmodalität ab, deren Bilder zum Trainieren der bereitgestellten Einheit verwendet worden sind. Wenn beispielsweise MR-Bilder zum Trainieren der Einheit verwendet worden sind, weist das mittels der trainierten Einheit rekonstruierte Ultraschallbild MR-Bildcharakteristika auf, das heißt, in dem mittels der trainierten Einheit erzeugten Ultraschallbild sind Charakteristika des Objektes sichtbar, die normalerweise nicht in einem Ultraschallbild sichtbar sind, sondern in einem MR-Bild.

Auch wenn das mittels der trainierten Einheit erzeugte Ultraschallbild beispielsweise MR-Bildcharakteristika aufweist, so ist es, wie oben erläutert, dennoch ein Ultraschallbild, das übliche Ultraschallbildeigenschaften wie einen Tiefenabfall aufweisen kann. Der Tiefenabfall bezieht sich beispielweise darauf, dass mit zunehmenden Abstand zu einer Ultraschallsonde und damit mit zunehmender Tiefe in einem Objekt, beispielsweise in einem Patienten, ein Ultraschallsignal aufgrund von beispielsweise Absorption und Streuung schwächer wird, wodurch auch das Signal-zu-Rausch-Verhältnis mit zunehmenden Tiefe abnimmt.

Das Ultraschallbild kann beispielsweise ein zweidimensionales oder ein dreidimensionales Bild sein. Die Ultraschalldatenbereitstellungseinheit kann eine Speichereinheit sein, in der die Ultraschallrohdaten gespeichert sind und die die gespeicherten Ultraschallrohdaten bereitstellen kann. Die Ultraschalldatenbereitstellungseinheit kann aber auch eine Empfangseinheit sein, die die Ultraschallrohdaten empfängt und die empfangenen Ultraschallrohdaten bereitstellt. Zudem kann die Ultraschalldatenbereitstellungseinheit auch die Ultraschallbildungsmodalität sein, mit der die Ultraschallrohdaten aufgenommen worden sind.

Die Trainierte-Einheit-Bereitstellungseinheit kann auch eine Speichereinheit sein, die in diesem Fall die trainierte Einheit bereitstellt. Die Trainierte-Einheit-Bereitstellungseinheit kann aber auch eine Empfangseinheit sein, die angepasst ist, die trainierte Einheit zu empfangen und die empfangene trainierte Einheit bereitzustellen.

Die trainierte Einheit ist eine Einheit, die mittels Maschinenlernens angepasst, das heißt trainiert, ist, auf Basis von Ultraschallrohdaten eines Objekts ein Ultraschallbild des Objekts zu erzeugen, das nicht der Ultraschallbildgebungsmodalität entspricht. Das heißt, die trainierte Einheit ist eine Einheit, die mit Ultraschallrohdaten der Ultraschallbildgebungsmodalität und mit Bildern einer anderen Bildgebungsmodalität trainiert worden ist. Die Trainierte-Einheit-Bereitstellungseinheit bzw. die bereitgestellte trainierte Einheit sind demnach auch über die Art des Trainings der trainierten Einheit definiert. Die Trainierte-Einheit-Bereitstellungseinheit ist eine Einheit, die angepasst ist, eine trainierte Einheit bereitzustellen, die mit a) Ultraschallrohdaten, die mit einer UltraschallBildgebungsmodalität aufgenommen worden sind, und b) Bildern trainiert worden ist, die mit einer anderen Bildgebungsmodalität aufgenommen worden sind. Diese anderen Bilder sind beispielsweise CT-Bilder, MR-Bilder, PET-Bilder, SPECT-Bilder, MPI-Bilder oder Bilder einer anderen Bildgebungsmodalität. Die bereitgestellte trainierte Einheit ist so trainiert worden, dass sie - bei Eingabe der Ultraschallrohdaten - diejenigen Bilder ausgibt, die mittels der anderen Bildgebungsmodalität erzeugt worden sind, wobei, da die ausgegebenen Bilder, wie oben erläutert, Ultraschallbilder sind und daher nicht exakt die Bilder, die mittels der anderen Bildgebungsmodalität erzeugt worden sind, dies bedeutet, dass mit dem Ziel trainiert wird, diese Bilder auszugeben, dies aber nicht erreicht wird. Mit anderen Worten, das Training wird so durchgeführt, dass Abweichungen zwischen dem mittels der zu trainierenden Einheit erzeugten Ausgangsbild und dem entsprechenden Trainingsbild minimiert werden.

Während des Trainings wird die Einheit wie beispielsweise ein neuronales Netz also mit dem Ziel trainiert, bei Eingabe der Ultraschallrohdaten, die mittels einer Ultraschallbildgebungsmodalität aufgenommen worden sind, ein Bild zu erzeugen, das einem Bild einer anderen Bildgebungsmodalität entspricht wie beispielsweise einer MR-Bildgebungsmodalität. Die trainierte Einheit, die als Eingang die Ultraschallrohdaten verwendet, wird als Ausgangsbild aber, wie oben beschrieben, nicht exakt das in diesem Beispiel verwendet MR-Bild erzeugen, sondern ein Ultraschallbild, das beispielsweise ein Speckle-Muster oder einen Tiefenabfall aufweist, mit in dem MR-Bild sichtbaren MR-Bildcharakteristika. Im Ergebnis wird die bereitgestellte trainierte Einheit demnach, wie ebenfalls oben erläutert, trotz des Ziels, beispielsweise das MR-Bild auszugeben, so trainiert, dass Abweichungen zwischen dem von der zu trainierenden Einheit auf Basis der Ultraschallrohdaten erzeugten Ultraschallbild und dem in diesem Beispiel zum Training verwendeten MR-Bild minimiert werden.

Der Begriff "Bildgebungsmodalität" bezeichnet eine entsprechende Klasse von Medizingeräten, die für bildgebende Verfahren in der medizinischen Diagnostik eingesetzt werden, wobei sich verschiedene Medizingeräte einer selben Bildgebungsmodalität bezüglich der grundlegenden Bildgebungstechniken gleichen. Eine Bildgebungsmodalität ist beispielsweise CT. Eine andere Bildgebungsmodalität ist beispielsweise MR. Eine weitere Bildgebungsmodalität ist beispielsweise Echo-Ultraschall. Wiederum eine andere Bildgebungsmodalität ist Transmissions-Ultraschall. Eine weitere Bildgebungsmodalität ist PET. Ferner sind weitere Bildgebungsmodalitäten SPECT und MPI.

Die Ultraschalldatenbereitstellungseinheit ist bevorzugt angepasst, als Ultraschallrohdaten Ultraschall-Echo-Rohdaten bereitzustellen. Das heißt, bevorzugt wird eine Ultraschall-Echo-Bildungsmodalität verwendet, um die Ultraschallrohdaten aufzunehmen. Das Ultraschallbild, das nicht dieser Ultraschall-Echo-Bildungsmodalität entspricht, ist dann ein Ultraschallbild, das Bild-Charakteristika aufweist, die keine Bild-Charakteristika einer Ultraschall-Echo-Bildungsmodalität sind, also einer bekannten Ultraschall-Echo-Bildungsmodalität. Dieses Bild weist bspw. CT-Bildcharakteristika, MR-Bildcharakteristika, SPECT-Bildcharakteristika, PET-Bildcharakteristika, MPI-Bildcharakteristika oder Bildcharakteristika einer anderen Bildgebungsmodalität auf. Das Ultraschallbild kann auch Transmissions-Ultraschallbild-Charakteristika aufweisen, die normalerweise in Bildern enthalten sind, die mit einer Ultraschall-Transmissions-Bildungsmodalität erzeugt werden. Beispielsweise kann das mit der trainierten Einheit erzeugte Ultraschallbild ein Schallgeschwindigkeitsbild sein. Zu diesem Zweck kann die trainierte Einheit mit Schallgeschwindigkeitsbildern trainiert werden, die mit Hilfe einer Ultraschall-Transmissions-Bildungsmodalität aufgenommen worden sind.

Eine Bildcharakteristik, die einen Fachmann sofort erkennen lässt, dass ein Ultraschallbild mit einer CT-Bildcharakteristik vorliegt, ist der charakteristische starke Kontrast zwischen Knochen und Gewebe, wobei die Knochen relativ hell und das Gewebe relativ dunkel dargestellt werden. Eine typische Bildcharakteristik, die einen Fachmann sofort erkennen lässt, dass ein Ultraschallbild mit einer MR-Bildcharakteristik vorliegt, bezieht sich auf den Gewebekontrast. Ein charakteristischer MR-Gewebekontrast ermöglicht beispielsweise die Unterscheidung verschiedener Gewebearten bzw. verschiedener Organe. Bei der Kopfbildgebung führt die für ein MR-Bild typische MR-Bildcharakteristik dazu, dass weiße Hirnsubstanz sehr gut von grauer Hirnsubstanz unterschieden werden kann. Bildcharakteristika, die einen Fachmann sofort erkennen lassen, dass ein Ultraschallbild mit Ultraschall-Echo-Bildcharakteristika vorliegt, sind beispielsweise Speckle-Muster, Abschattungen entlang der Schallrichtung und eine tiefenabhängige Signalintensität.

Wenn als Ultraschallrohdaten Ultraschall-Echo-Rohdaten verwendet werden, können mittels der trainierten Einheit Ultraschallbilder mit Bildcharakteristika einer anderen Bildgebungsmodalität mit weiter verbesserter Bildqualität und verbessertem Kontrast erzeugt werden.

Ultraschallrohdaten sind Ultraschalldaten, die kein Ultraschallbild bilden. Die Ultraschallrohdaten sind beispielsweise unmittelbar gemessene Ultraschalldaten oder Ultraschalldaten, die verarbeitet, beispielsweise gefiltert worden sind, ohne dass diese Verarbeitung eine Bilderzeugung beinhaltet. Insbesondere sind Ultraschallrohdaten Messdaten einzelner Elemente eines Ultraschallempfangsarrays, das null-, ein- oder zweidimensional sein kann. Ultraschallrohdaten können nur aus den Frequenzanteilen des Sendeultraschalls bestehen oder auch aus frequenzverschobenen ("Dopplerverschiebung") oder durch nichtlineare Prozesse erzeugte höheren harmonischen Frequenzanteilen ("Harmonic frequencies").

Außerdem können Ultraschallrohdaten mit zusätzlicher Präparation erzeugt werden. Dies beinhaltet die Messung von Scherwellen für eine Ultraschall-Elastographie-Messung oder auch besondere Sende-Pulsformen und Kombinationen wie zum Beispiel Pulsinversion oder Pulskodierung.

Die Trainierte-Einheit-Bereitstellungseinheit ist bevorzugt angepasst, als die mittels Maschinenlernens trainierte Einheit ein neuronales Netz bereitzustellen, das angepasst ist, auf Basis von Ultraschallrohdaten eines Objekts ein Ultraschall-Bild des Objekts zu erzeugen, das nicht der Ultraschallbildgebungsmodalität entspricht, das zur Aufnahme der Ultraschallrohdaten verwendet worden ist. Insbesondere ist die Trainierte-Einheit-Bereitstellungseinheit angepasst, als das neuronale Netz ein Deep-Learning-Netz bereitzustellen. Die Verwendung eines neuronalen Deep-Learning-Netzes kann zu einer weiter verbesserten Bildqualität und mehr Informationsgehalt in dem Bild führen. Die Trainierte-Einheit-Bereitstellungseinheit kann angepasst sein, als das neuronale Netz ein nicht vollständig faltendes neuronales Netz, das heißt ein nicht vollständiges Convolutional Neural Network (CNN), bereitzustellen. Die Verwendung eines derartigen nicht vollständig faltenden neuronalen Netzes führt dazu, dass die Erzeugung des Ultraschallbildes auf Basis des neuronalen Netzes nicht oder zumindest nicht ausschließlich auf einer Mustererkennung beruht. Dies kann zu einer weiter verbesserten Qualität des erzeugten Ultraschallbildes führen. Das nicht vollständig faltende neuronale Netz weist zumindest eine Ebene ("layer") auf, die keine Faltungsoperationen durchführt. In einer Ausführungsform weist das nicht vollständig faltende neuronale Netz nur eine Ebene auf, die Faltungsoperationen durchführt. In einer weiteren Ausführungsform weist das nicht vollständige faltende neuronale Netz mehrere Ebenen auf, die Faltungsoperationen durchführen, wobei die Anzahl (erste Anzahl) der Ebenen, die Faltungsoperationen durchführen, geringer ist als die Anzahl (zweite Anzahl) der Ebenen, die keine Faltungsoperationen durchführen. Bevorzugt umfasst die erste Anzahl 30 % oder weniger und weiter bevorzugt 20 % oder weniger der Ebenen des nicht vollständig faltendenden neuronalen Netzes. Dementsprechend umfasst die zweite Anzahl bevorzugt 70 % oder mehr und weiter bevorzugt 80 % oder mehr der Ebenen des nicht vollständig faltendenden neuronalen Netzes. Zudem umfasst in einer bevorzugten Ausführungsform die erste Anzahl nur ein oder zwei Ebenen des nicht vollständig faltendenden neuronalen Netzes und die zweite Anzahl die restlichen Ebenen des nicht vollständig faltendenden neuronalen Netzes, das heißt, die Gesamtanzahl der Ebenen des nicht vollständig faltendenden neuronalen Netzes minus 1 bzw. minus 2. Die Ebenen, die keine Faltungsoperationen durchführen, können beispielsweise voll verbundene Ebenen (engl. "fully connected layers") und/oder Restricted Boltzmann Machines ("RBM") umfassen. In einer Ausführungsform bestehen die Ebenen, die keine Faltungsoperationen durchführen, nur aus voll verbundene Ebenen (engl. "fully connected layers") und/oder Restricted Boltzmann Machines ("RBM")

Das Ultraschall-Bilderzeugungssystem kann zudem eine Bild-Bereitstellungseinheit zum Bereitstellen eines weiteren Bildes des Objekts aufweisen, das nicht der Ultraschallbildgebungsmodalität entspricht, wobei dieses weitere Bild nicht unter Verwendung der trainierten Einheit erzeugt worden ist. Außerdem kann das Ultraschall-Bilderzeugungssystem eine Registrierungseinheit aufweisen, die angepasst ist, das bereitgestellte weitere Bild und das unter Verwendung der trainierten Einheit erzeugte Ultraschallbild miteinander zu registrieren. Die Registrierungseinheit kann angepasst sein, das bereitgestellte weitere Bild derart zu transformieren, dass es mit dem unter Verwendung der trainierten Einheit erzeugten Ultraschallbild registriert ist. Das bereitgestellte weitere Bild ist bevorzugt ein bereits zuvor mit einer anderen Bildgebungsmodalität aufgenommenes Bild, das in einer Speichereinheit abgelegt worden ist, wobei die Speichereinheit dieses zuvor aufgenommene Bild bereitstellt. Diese Speichereinheit könnte daher als Bild-Bereitstellungseinheit aufgefasst werden. Dieses zuvor aufgenommene Bild ist beispielsweise ein CT-Bild, ein MR-Bild, ein PET-Bild, ein SPECT-Bild, ein MPI-Bild oder ein Bild einer anderen Bildgebungsmodalität, die sich von der Ultraschallbildgebungsmodalität unterscheidet, die zur Aufnahme der Ultraschallrohdaten verwendet worden ist. Das zuvor aufgenommene Bild, das bevorzugt nicht auf Ultraschallrohdaten basiert, sondern beispielsweise auf CT-Rohdaten oder MR-Rohdaten, kann eine noch bessere Bildqualität aufweisen, als das unter Verwendung der trainierten Einheit auf Basis der Ultraschallrohdaten erzeugte Ultraschallbild. Durch Transformieren des weiteren bereitgestellten Bildes, das heißt, beispielsweise des CT-Bildes oder des MR-Bildes, derart, dass es mit dem unter Verwendung der trainierten Einheit und der Ultraschallrohdaten erzeugten Ultraschallbildes registriert ist, kann ein Bild des Objekts bereitgestellt werden, das eine weiter verbesserte Bildqualität aufweisen kann.

Das Ultraschall-Bilderzeugungssystem kann eine Elementbestimmungseinheit und eine Indikatorerzeugungseinheit aufweisen, wobei die Elementbestimmungseinheit angepasst ist, einen Ort eines Elements in dem unter Verwendung der trainierten Einheit erzeugten Bild zu bestimmen, und wobei die Indikatorerzeugungseinheit angepasst ist, einen Indikator, der den Ort des Elements in dem weiteren bereitgestellten Bild anzeigt, auf Basis der Registrierung und des bestimmten Ortes zu erzeugen. Das Element kann insbesondere ein interventionelles Instrument sein, wie beispielsweise ein Katheter oder eine Nadel, wobei das weitere bereitgestellte Bild ein Bild sein kann, das aufgenommen worden ist, bevor das interventionelle Instrument in den Körper eingeführt worden ist. Das weitere bereitgestellte Bild kann demnach ein präinterventionelles Bild sein. Mithilfe der Elementbestimmungseinheit und der Indikatorerzeugungseinheit kann in dem Ultraschallbild, das unter Verwendung der trainierten Einheit und der Ultraschallrohdaten erzeugt worden ist und das ein interventionelles Bild sein kann, das interventionelle Instrument detektiert, insbesondere segmentiert werden, um den Ort des Instruments im Körper festzustellen, wonach dieser Ort in dem präinterventionellen Bild, das beispielsweise ein CT-Bild oder ein MR-Bild ist, angezeigt werden kann. Dies kann beispielsweise eine verbesserte Führung eines Chirurgen ermöglichen, während er das interventionelle Instrument innerhalb des Körpers bewegt.

Die Registrierungseinheit ist bevorzugt angepasst, zur Registrierung eine elastische Transformation durchzuführen. Die Verwendung einer elastischen Transformation kann zu einer weiter verbesserten Registrierung, insbesondere zu einer weiter verbesserten Transformation, des unter Verwendung der trainierten Einheit und auf Basis der Ultraschallrohdaten erzeugten Ultraschallbildes führen. Dies wiederum kann eine weiter verbesserte Bildqualität ermöglichen. Auch die oben beschriebene Führung eines Chirurgen während eines interventionellen Eingriffes kann hierdurch verbessert werden.

Bevorzugt sind die Bild-Bereitstellungseinheit und die Bilderzeugungseinheit derart angepasst, dass das bereitgestellte weitere Bild und das unter Verwendung der trainierten Einheit erzeugte Ultraschallbild derselben Bildgebungsmodalität entsprechen. Das heißt, das mittels der trainierten Einheit erzeugte Ultraschallbild weist Bildcharakteristika auf, die der Bildgebungsmodalität entsprechen, mit der das bereitgestellte weitere Bild aufgenommen worden ist. In anderen Worten, in diesem Beispiel wurden zum Training der Einheit Bilder einer Bildgebungsmodalität verwendet, mit der auch das weitere bereitgestellte Bild aufgenommen worden ist. Beispielsweise wurden zum Trainieren der Einheit MR-Bilder verwendet und das bereitgestellte weitere Bild ist ein MR-Bild. Auch dies kann zu einer verbesserten Registrierung und damit beispielsweise zu einer weiter verbesserten Bildqualität und/oder einer verbesserten Führung eines Chirurgen während eines interventionellen Eingriffs führen.

Das Ultraschall-Bilderzeugungssystem kann eine Trainingseinheit zum Trainieren der Einheit aufweisen, wobei die Trainingseinheit angepasst ist, die Einheit derart zu trainieren, dass die trainierte Einheit bei Eingabe der bereitgestellten Ultraschallrohdaten des Objekts das bereitgestellte Bild ausgibt, wobei, da die ausgegebenen Bilder, wie oben erläutert, Ultraschallbilder sind und daher nicht exakt die Bilder, die mittels einer anderen Bildgebungsmodalität erzeugt worden sind, dies bedeutet, dass mit dem Ziel trainiert wird, das bereitgestellte Bild auszugeben, dies aber nicht erreicht wird. Mit anderen Worten, das Training wird so durchgeführt, dass Abweichungen zwischen dem mittels der zu trainierenden Einheit erzeugten Ausgangsbild und dem entsprechenden Trainingsbild minimiert werden.

Das Ultraschall-Bilderzeugungssystem kann demnach nicht nur dazu verwendet werden, beispielsweise auf Basis von Ultraschallrohdaten ein Bild mit CT-Bildcharakteristika oder mit MR-Bildcharakteristika zu erzeugen, sondern auch dazu, die trainierte Einheit weiter zu trainieren. Dieses weitere Training kann zu einer verbesserten mittels Maschinenlernens trainierten Einheit und damit auch zu einer weiter verbesserten Bildqualität der beispielsweise auf Basis der Ultraschallrohdaten erzeugten Bilder mit CT-Bildcharakteristika oder MR-Bildcharakteristika führen.

Die Trainingseinheit versucht also, die Einheit derart zu trainieren, dass die trainierte Einheit bei Eingabe der bereitgestellten Ultraschallrohdaten des Objekts das jeweilige bereitgestellte Bild einer anderen Bildgebungsmodalität wie beispielsweise ein MR-Bild ausgibt. Obwohl das Training mit diesem Ziel durchgeführt wird, wird sich, wie oben ausgeführt, das mittels der trainierten Einheit erzeugte Ultraschallbild von einem beispielsweise zum Training verwendeten MR-Bild unterscheiden. Diese Unterscheidung basiert beispielsweise darauf, dass das mittels der trainierten Einheit erzeugte Ultraschallbild Ultraschallbildcharakteristika aufweisen kann wie den oben erwähnten Tiefenabfall. Die Trainingseinheit ist angepasst, die Einheit derart zu trainieren, dass Abweichungen des mittels der trainierten Einheit erzeugten Ultraschallbildes von dem zum Training verwendeten Bild einer anderen Bildgebungsmodalität minimiert werden, um ein Ultraschallbild zu erzeugen, das möglichst gut dem zum Training verwendeten Bild der anderen Bildgebungsmodalität entspricht.

Zudem wird die oben genannte Aufgabe durch ein Trainingssystem zum Trainieren einer Einheit mittels Maschinenlernens gelöst, wobei das Trainingssystem aufweist:
- eine Ultraschalldatenbereitstellungseinheit zum Bereitstellen von Ultraschallrohdaten eines Objekts, die mit einer Ultraschallbildungsmodalität aufgenommen worden sind oder die durch Simulation einer Aufnahme mit der Ultraschallbildungsmodalität erzeugt worden sind,
- eine Zu-Trainierende-Einheit-Bereitstellungseinheit zum Bereitstellen einer mittels Maschinenlernens zu trainierenden Einheit,
- eine Bild-Bereitstellungseinheit zum Bereitstellen eines Bildes des Objekts, das nicht mit der Ultraschallbildungsmodalität aufgenommen worden ist, mit der die Ultraschallrohdaten aufgenommen worden sind, und nicht durch Simulation einer Aufnahme mit der Ultraschallbildungsmodalität erzeugt worden ist,
- eine Trainingseinheit zum Trainieren der bereitgestellten zu trainierenden Einheit derart, dass die trainierte Einheit bei Eingabe der bereitgestellten Ultraschallrohdaten des Objekts das bereitgestellte Bild ausgibt, wobei, da die ausgegebenen Bilder, wie oben erläutert, Ultraschallbilder sind und daher nicht exakt die Bilder, die mittels der anderen Bildgebungsmodalität erzeugt worden sind, dies bedeutet, dass mit dem Ziel trainiert wird, diese Bilder auszugeben, dies aber nicht erreicht wird. Mit anderen Worten, das Training wird so durchgeführt, dass Abweichungen zwischen dem mittels der zu trainierenden Einheit erzeugten Ausgangsbild und dem entsprechenden Trainingsbild minimiert werden.

Die Trainingseinheit ist also, wie oben bereits ausgeführt, so ausgebildet, dass Abweichungen des mittels der trainierten Einheit erzeugten Ultraschallbildes von dem bereitgestellten Bild, das beispielsweise ein MR-Bild ist, minimiert werden. Die derart optimierte trainierte Einheit wird dann ein Ultraschallbild auf Basis von Ultraschallrohdaten erzeugen, das Charakteristika der Bildgebungsmodalität aufweist, mit der das bereitgestellte Bild, das zum Training verwendet worden ist, erzeugt worden ist.

Die Bild-Bereitstellungseinheit kann eine Speichereinheit sein, in der das Bild des Objekts gespeichert ist und das angepasst ist, das gespeicherte Bild bereitzustellen. Die Bild-Bereitstellungseinheit kann auch eine Empfangseinheit sein, die angepasst ist, das Bild zu empfangen und das empfangene Bild bereitzustellen. Zudem kann die Bild-Bereitstellungseinheit auch eine Mess- bzw. Aufnahmeeinheit sein, die angepasst ist, das Bild aufzunehmen und das aufgenommen Bild bereitzustellen. Beispielsweise kann die Bild-Bereitstellungseinheit ein CT-System oder ein MR-System sein. Das bereitgestellte Bild ist beispielsweise ein CT-Bild, ein MR-Bild oder ein Bild einer anderen Bildgebungsmodalität, die nicht die Bildgebungsmodalität ist, mit der die Ultraschallrohdaten aufgenommen worden sind wie beispielsweise ein PET-Bild, ein SPECT-Bild oder ein MPI-Bild.

Es ist bevorzugt, dass die Ultraschalldatenbereitstellungseinheit und die Bild-Bereitstellungseinheit angepasst sind, die Ultraschallrohdaten und das bereitgestellte Bild von einem selben Bereich des Objekts, insbesondere gleichzeitig, aufzunehmen. Durch eine gleichzeitige Aufnahme der Ultraschallrohdaten und des bereitgestellten Bildes und durch die Verwendung dieser gleichzeitig aufgenommenen Ultraschallrohdaten und des Bildes zum Trainieren der Einheit wie beispielsweise eines neuronalen Netzes kann eine Beeinträchtigung der Qualität des Trainings der Einheit durch Bewegungen des Objekts verhindert werden. Dies kann zu einer verbesserten, schließlich trainierten Einheit und damit zu einer weiter verbesserten Bildqualität führen, wenn die trainierte Einheit dazu verwendet wird, auf Basis von Ultraschallrohdaten beispielsweise ein Ultraschallbild mit MR-Bildcharakteristika zu erzeugen.

Es ist weiter bevorzugt, dass die Trainingseinheit angepasst ist, a) zunächst auf Basis der bereitgestellten Ultraschallrohdaten ein Ultraschallbild, insbesondere ein Ultraschalltomographiebild, des Objekts zu erzeugen, ohne hierzu die zu trainierende Einheit zu verwenden, b) dann die Einheit derart zu trainieren, dass die trainierte Einheit bei Eingabe der bereitgestellten Ultraschallrohdaten des Objekts das erzeugte Ultraschall-Bild ausgibt, und c) schließlich die trainierte Einheit weiter derart zu trainieren, dass die trainierte Einheit bei Eingabe der bereitgestellten Ultraschallrohdaten des Objekts das bereitgestellte Bild ausgibt, das von der Bild-Bereitstellungseinheit bereitgestellt worden ist und insbesondere ein Tomographiebild ist, wobei, da die ausgegebenen Bilder, wie oben erläutert, Ultraschallbilder sind und daher nicht exakt die Bilder, die mittels der anderen Bildgebungsmodalität erzeugt worden sind, dies bedeutet, dass mit dem Ziel trainiert wird, diese Bilder auszugeben, dies aber nicht erreicht wird. Mit anderen Worten, das Training wird so durchgeführt, dass Abweichungen zwischen dem mittels der zu trainierenden Einheit erzeugten Ausgangsbild und dem entsprechenden Trainingsbild minimiert werden.

Es kann demnach vorgesehen sein, die Einheit zweistufig zu trainieren, wobei in einer ersten Stufe die geometrische Abbildung trainiert wird und in einer zweiten Stufe die Kontrastabbildung, das heißt, die Abbildung von der Ultraschallbildgebungsmodalität zu einer anderen Bildgebungsmodalität. Diese Zweistufigkeit kann zu einer weiter verbesserten, trainierten Einheit und damit, wenn die trainierte Einheit zur Bilderzeugung verwendet wird, zu einer weiter verbesserten Qualität des Ultraschallbildes mit beispielsweise CT-Bildcharakteristika oder MR-Bildcharakteristika führen, das auf Basis von Ultraschallrohdaten erzeugt wird. Diese Zweistufigkeit kann auch zu einem schnelleren Trainingserfolg führen.

Die Ultraschalldatenbereitstellungseinheit kann angepasst sein, Ultraschall-Echo-Rohdaten und Ultraschall-Transmissions-Rohdaten als Ultraschallrohdaten bereitzustellen. Zudem kann die Trainingseinheit angepasst ein, a) zunächst auf Basis der bereitgestellten Ultraschall-Transmissions-Rohdaten ein Ultraschalltomographiebild des Objekts zu erzeugen, ohne hierzu die zu trainierende Einheit zu verwenden, b) dann die Einheit derart zu trainieren, dass die trainierte Einheit bei Eingabe der bereitgestellten Ultraschall-Transmissions-Rohdaten des Objekts das erzeugte Ultraschalltomographiebild ausgibt, und c) schließlich die trainierte Einheit weiter derart zu trainieren, dass die trainierte Einheit bei Eingabe der bereitgestellten Ultraschall-Echo-Rohdaten des Objekts das bereitgestellte Bild ausgibt, das von der Bild-Bereitstellungseinheit bereitgestellt worden ist, wobei das bereitgestellte Bild bevorzugt ein Tomographiebild ist und wobei, da die ausgegebenen Bilder, wie oben erläutert, Ultraschallbilder sind und daher nicht exakt die Bilder, die mittels der anderen Bildgebungsmodalität erzeugt worden sind, dies auch hier bedeutet, dass mit dem Ziel trainiert wird, diese Bilder auszugeben, dies aber nicht erreicht wird. Mit anderen Worten, das Training wird so durchgeführt, dass Abweichungen zwischen dem mittels der zu trainierenden Einheit erzeugten Ausgangsbild und dem entsprechenden Trainingsbild minimiert werden.

Die Erzeugung des Ultraschalltomographiebildes auf Basis der Ultraschall-Transmissions-Rohdaten kann beispielsweise eine bekannte Rekonstruktion sein, die insbesondere nur die Ultraschall-Transmissions-Rohdaten verwenden. Die Erzeugung des Ultraschalltomographiebildes auf Basis der Ultraschall-Transmissions-Rohdaten kann aber auch eine Rekonstruktion sein, die zusätzlich die Ultraschall-Echo-Rohdaten verwendet, wobei die Ultraschall-Transmissions-Rohdaten verwendet werden, um die Schallgeschwindigkeit, insbesondere eine Schallgeschwindigkeitskarte, innerhalb des Objekts zu bestimmen, wobei dann das Ultraschalltomographiebild auf bekannte Weise mittels den Ultraschall-Echo-Rohdaten unter Berücksichtigung der bestimmten Schallgeschwindigkeit rekonstruiert wird.

Wenn im Rahmen des zweistufigen Trainings in der ersten Stufe bereits mit einem Ultraschalltomographiebild die geometrische Abbildung trainiert wird und wenn während der zweiten Stufe mit einem tomographischen, bereitgestellten Bild die Kontrastübertragung trainiert wird, das heißt, wenn in beiden Stufen mit Tomographiebildern trainiert wird, kann das Training der trainierten Einheit weiter verbessert werden. Dies wiederum kann schließlich zu einer weiter verbesserten Bildqualität führen, wenn die verbesserte trainierte Einheit zum Erzeugen beispielsweise eines Ultraschallbildes mit CT-Bildcharakteristika oder MR-Bildcharakteristika auf Basis von Ultraschallrohdaten verwendet wird.

Zudem wird die oben genannte Aufgabe durch eine mittels Maschinenlernens trainierte Einheit gelöst, die angepasst ist, auf Basis von Ultraschallrohdaten eines Objekts ein Ultraschall-Bild des Objekts zu erzeugen, das nicht der Ultraschallbildgebungsmodalität entspricht, das zur Aufnahme der Ultraschallrohdaten verwendet worden ist.

Weiterhin wird die oben genannte Aufgabe durch ein Ultraschall-Bilderzeugungsverfahren zum Erzeugen eines Bildes eines Objekts gelöst, wobei das Bilderzeugungsverfahren aufweist:
- Bereitstellen von Ultraschallrohdaten des Objekts, die mit einer Ultraschallbildungsmodalität aufgenommen worden sind, mittels einer Ultraschalldatenbereitstellungseinheit,
- Bereitstellen einer mittels Maschinenlernens trainierten Einheit, die angepasst ist, auf Basis von Ultraschallrohdaten eines Objekts ein Ultraschall-Bild des Objekts zu erzeugen, das nicht der Ultraschallbildgebungsmodalität entspricht, mit der die Ultraschallrohdaten aufgenommen worden sind, mittels einer Trainierte-Einheit-Bereitstellungseinheit und
- Erzeugen des Bildes des Objekts unter Verwendung der bereitgestellten trainierten Einheit auf Basis der bereitgestellten Ultraschallrohdaten des Objekts mittels einer Bilderzeugungseinheit.

Des Weiteren wird die oben genannte Aufgabe durch ein Trainingsverfahren zum Trainieren einer Einheit mittels Maschinenlernens gelöst, wobei das Trainingsverfahren aufweist:
- Bereitstellen von Ultraschallrohdaten eines Objekts mittels einer Ultraschalldatenbereitstellungseinheit, die mit einer Ultraschallbildungsmodalität aufgenommen worden sind oder die durch Simulation einer Aufnahme mit der Ultraschallbildungsmodalität erzeugt worden sind,
- Bereitstellen einer zu trainierenden Einheit mittels einer Trainierte-Einheit-Bereitstellungseinheit,
- Bereitstellen eines Bildes des Objekts, das nicht mit der Ultraschallbildungsmodalität aufgenommen worden ist, mit der die Ultraschallrohdaten aufgenommen worden sind, und nicht durch Simulation einer Aufnahme mit der Ultraschallbildungsmodalität erzeugt worden ist, mittels einer Bild-Bereitstellungseinheit,
- Trainieren der bereitgestellten Einheit derart, dass die trainierte Einheit bei Eingabe der bereitgestellten Ultraschallrohdaten des Objekts das bereitgestellte Bild ausgibt, mittels einer Trainingseinheit, wobei, da die ausgegebenen Bilder, wie oben erläutert, Ultraschallbilder sind und daher nicht exakt die Bilder, die mittels der anderen Bildgebungsmodalität erzeugt worden sind, dies bedeutet, dass mit dem Ziel trainiert wird, das

bereitgestellte Bild auszugeben, dies aber nicht erreicht wird. Mit anderen Worten, das Training wird so durchgeführt, dass Abweichungen zwischen dem mittels der zu trainierenden Einheit erzeugten Ausgangsbild und dem entsprechenden Trainingsbild minimiert werden.

Außerdem wird die oben genannte Aufgabe durch ein Computerprogramm zum Erzeugen eines Bildes eines Objekts gelöst, wobei das Computerprogramm angepasst ist, das Ultraschall-Bilderzeugungsverfahren gemäß Anspruch 15 durchzuführen, wenn es auf einem Ultraschall-Bilderzeugungssystem zum Erzeugen von Bildern gemäß einem der Ansprüche 1 bis 8 ausgeführt wird.

Zudem wird die oben genannte Aufgabe durch ein Computerprogramm zum Trainieren einer Einheit mittels Maschinenlernens gelöst, wobei das Computerprogramm angepasst ist, das Trainingsverfahren zum Trainieren einer Einheit mittels Maschinenlernens gemäß Anspruch 15 durchzuführen, wenn es auf einem Trainingssystem zum Trainieren einer Einheit mittels Maschinenlernens gemäß einem der Ansprüche 9 bis 13 ausgeführt wird.

Es sollte verstanden werden, dass das Ultraschall-Bilderzeugungssystem nach Anspruch 1, das Trainingssystem nach Anspruch 9, das Ultraschall-Bilderzeugungsverfahren nach Anspruch 15, das Trainingsverfahren nach Anspruch 16 und die Computerprogramme nach den Ansprüchen 17 und 18 ähnliche und/oder identische bevorzugte Ausführungsformen aufwiesen, wie sie insbesondere in den abhängigen Ansprüchen definiert sind.

Im Folgenden werden Ausführungsformen der Erfindung unter Bezugnahme auf folgende Figuren beschrieben, wobei
- Fig. 1: schematisch und exemplarisch eine Ausführungsform eines Ultraschall-Bilderzeugungssystems zum Erzeugen eines Bildes eines Objekts zeigt,
- Fig. 2: schematisch und exemplarisch eine Ausführungsform eines Trainingssystems zum Trainieren eines neuronalen Netzes zeigt,
- Fig. 3: schematisch und exemplarisch Details einer Rohdatenaufnahmeeinheit des Trainingssystems zeigt,
- Fig. 4: ein Flussdiagramm zeigt, das eine Ausführungsform eines Ultraschall-Bilderzeugungsverfahrens zum Erzeugen eines Bildes eines Objekts illustriert, und
- Fig. 5: ein Flussdiagramm zeigt, das eine Ausführungsform eines Trainingsverfahrens zum Trainieren eines neuronalen Netzes illustriert.

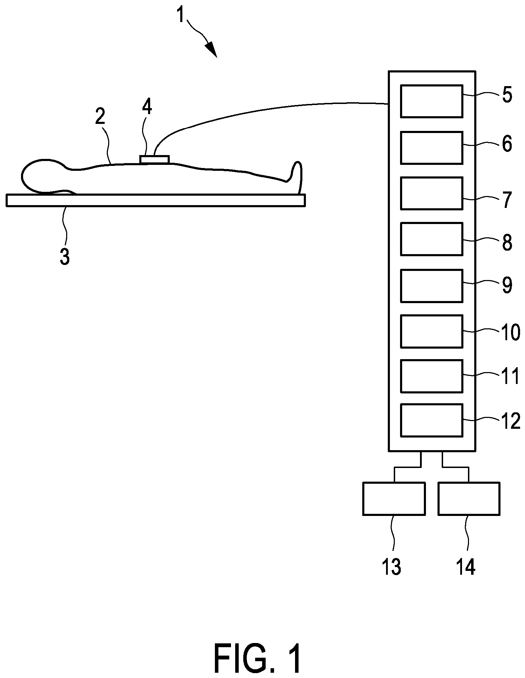

Eine Ausführungsform eines Ultraschall-Bilderzeugungssystems zum Erzeugen eines Bildes eines Patienten ist schematisch und beispielhaft in Fig. 1 gezeigt. Das Ultraschall-Bilderzeugungssystem 1 umfasst ein Ultraschallmessgerät 4, das heißt eine Ultraschallbildgebungsmodalität, und eine Steuerungseinheit 5 zum Steuern des Ultraschallmessgeräts 4. Mithilfe des Ultraschallmessgeräts 4 und der Steuerungseinheit 5 kann beispielsweise ein Arzt Ultraschallrohdaten des Patienten 2 aufnehmen, der sich auf einem Patiententisch 3 befindet. In dieser Ausführungsform sind das Ultraschallmessgerät 4 und die Steuerungseinheit 5 angepasst, Ultraschall-Echo-Rohdaten aufzunehmen.

Das Ultraschallmessgerät 4 kann beispielsweise ein Gerät sein, das von dem Arzt während der Aufnahme der Ultraschallrohdaten in der Hand gehalten werden kann (engl. *"handheld device"*). Das Ultraschallmessgerät kann aber natürlich auch ein stationäres Messgerät sein. Zudem kann das Ultraschallmessgerät, nachdem es beispielsweise von einem Arzt auf dem Patienten 2 platziert worden ist, losgelassen werden, so dass es während der Aufnahme der Ultraschallrohdaten nicht in der Hand gehalten wird.

Da mittels des Ultraschallmessgeräts 4 und der Steuerungseinheit 5 Ultraschallrohdaten bereitgestellt werden können, können das Ultraschallmessgerät 4 und die Steuerungseinheit 5 als Komponenten einer Ultraschalldatenbereitstellungseinheit zum Bereitstellen von Ultraschallrohdaten des Patienten 2 aufgefasst werden.

Das Ultraschall-Bilderzeugungssystem 1 umfasst des Weiteren eine Trainierte-Einheit-Bereitstellungseinheit 6 zum Bereitstellen einer mittels Maschinenlernens erzeugten Einheit, die in diesem Beispiel ein neuronales Netz ist, das angepasst ist, auf Basis von Ultraschallrohdaten eines Patienten ein Bild des Patienten zu erzeugen, das nicht der Ultraschall-Echo-Bildgebungsmodalität 4 entspricht. Die Trainierte-Einheit-Bereitstellungseinheit kann daher in diesem Beispiel auch als Neuronales-Netz-Bereitstellungseinheit bezeichnet werden. In dieser Ausführungsform ist die Trainierte-Einheit-Bereitstellungseinheit 6 angepasst, ein Ultraschallbild mit MR-Bildcharakteristika, beispielsweise mit T1-gewichtete-MR-Bildcharakteristika, auf Basis von Ultraschallrohdaten zu erzeugen, wobei das neuronale Netz ein Deep-Learning-Netz ist, insbesondere ein nicht vollständig faltendes neuronales Netz. Zudem umfasst das Ultraschall-Bilderzeugungssystem 1 eine Bilderzeugungseinheit 7 zum Erzeugen des Ultraschallbildes mit MR-Bildcharakteristika des Patienten 2 unter Verwendung des bereitgestellten neuronalen Netzes auf Basis der gemessenen Ultraschallrohdaten.

Das Ultraschall-Bilderzeugungssystem 1 umfasst bevorzugt auch eine Bild-Bereitstellungseinheit 8 zum Bereitstellen eines weiteren Bildes des Patienten 2, das nicht der Ultraschall-Echo-Bildgebungsmodalität 4 entspricht, wobei dieses Bild nicht unter Verwendung des neuronalen Netzes erzeugt worden ist. In dieser Ausführungsform ist die Bild-Bereitstellungseinheit 8 angepasst, ein MR-Bild des Patienten bereitzustellen, das zuvor, beispielsweise bereits vor ein paar Tagen, auf Basis von MR-Rohdaten des Patienten mithilfe bekannter Rekonstruktionsalgorithmen erzeugt worden ist. Diese bekannten Rekonstruktionsalgorithmen beinhalte beispielsweise eine Fourier-Transformation von einem k-Raum in einen Ortsraum. Dieses zuvor aufgenommene MR-Bild kann mittels einer Registrierungseinheit 9 mit dem von der Bilderzeugungseinheit 7 erzeugten Bild registriert werden, wobei das zuvor aufgenommene MR-Bild insbesondere mittels einer elastischen Transformation transformiert werden kann. Es kann demnach ein transformiertes, zuvor aufgenommenes MR-Bild durch Registrierung mit dem von der Bilderzeugungseinheit 7 erzeugten Bild erzeugt werden.

Zudem kann das Ultraschall-Bilderzeugungssystem 1 eine Elementbestimmungseinheit 10 aufweisen, die angepasst ist, einen Ort eines Elementes in dem unter Verwendung des neuronalen Netzes erzeugten Ultraschallbild mit MR-Bild-Charakteristika zu bestimmen. Die Elementbestimmungseinheit 10 kann hierzu beispielsweise bekannte Segmentierungsalgorithmen verwenden und das Element ist beispielsweise ein interventionelles Instrument, das während eines chirurgischen Eingriffes verwendet wird.

Dieses interventionelle Instrument kann in dem auf Basis der Ultraschallrohdaten erzeugten Ultraschallbild mit MR-Bildcharakteristika detektiert werden, um den Ort des interventionellen Instrumentes innerhalb dieses Bildes festzustellen. Durch die Registrierung dieses Ultraschallbildes mit MR-Bildcharakteristika mit dem zuvor aufgenommenen MR-Bild, das auf Basis von MR-Rohdaten erzeugt worden ist, kann der Ort des interventionellen Instrumentes auch in dem zuvor aufgenommenen MR-Bild gezeigt werden. Wenn das zuvor aufgenommene MR-Bild eine bessere Bildqualität aufweist als das auf Basis der Ultraschallrohdaten erzeugte Ultraschallbild mit MR-Bildcharakteristika, so kann das Anzeigen des Ortes des interventionellen Instrumentes in dem *"echten"* MR-Bild, das auf Basis der MR-Rohdaten erzeugt worden ist, eine Führung eines Chirurgen, der das interventionelle Instrument innerhalb des Patienten 2 bewegt, verbessern.

Zum Anzeigen des Ortes des interventionellen Instrumentes in dem MR-Bild, das auf Basis der MR-Rohdaten zuvor erzeugt worden ist, kann eine Indikatorerzeugungseinheit 11 verwendet werden, die angepasst ist, einen Indikator, der den Ort des interventionellen Instruments in dem zuvor aufgenommenen MR-Bild, das auf Basis von MR-Rohdaten präinterventionell aufgenommen worden ist, auf Basis der Registrierung und des bestimmten Ortes zu erzeugen.

Das Ultraschall-Bilderzeugungssystem 1 umfasst des Weiteren eine Trainingseinheit 12 zum Trainieren des neuronalen Netzes derart, dass das trainierte neuronale Netz bei Eingabe der bereitgestellten Ultraschallrohdaten des Patienten das von der Bild-Bereitstellungseinheit 8 erzeugte MR-Bild ausgibt. Das heißt, das Ultraschall-Bilderzeugungssystem 1 kann nicht nur angepasst sein, auf Basis von Ultraschallrohdaten unter Verwendung des neuronalen Netzes beispielsweise ein Ultraschallbild mit MR-Bildcharakteristika zu erzeugen, sondern es kann auch angepasst sein, das neuronale Netz weiter zu trainieren, um dies weiter zu verbessern. Die Trainingseinheit versucht, das neuronale Netz derart zu trainieren, dass bei Eingabe der Ultraschallrohdaten das MR-Bild ausgegeben wird. Da das von dem neuronalen Netz erzeugte Bild aber ein Ultraschallbild ist, ist klar, dass dies nur näherungsweise möglich ist. Die Trainingseinheit 12 ist daher angepasst, das neuronale Netz derart zu trainieren, dass Abweichungen des mittels des neuronalen Netzes erzeugten Ultraschallbildes von dem MR-Bild minimiert werden. Der Ausdruck "Trainieren des neuronalen Netzes derart, dass das trainierte neuronale Netz bei Eingabe der bereitgestellten Ultraschallrohdaten des Patienten das [...] MR-Bild ausgibt" bedeutet daher, dass die Trainingseinheit 12 das neuronale Netz mit diesem Ziel trainiert, indem entsprechende Abweichungen minimiert werden, wobei das von dem schließlich trainierten neuronalen Netz ausgegebene Ultraschallbild nicht exakt gleich dem MR-Bild ist, sondern diesem möglichst gut entspricht.

Das Ultraschall-Bilderzeugungssystem 1 umfasst des Weiteren eine Eingabeeinheit 13, wie beispielsweise eine Tastatur, eine Computermaus, ein berührungssensitives Display, et cetera und eine Ausgabeeinheit 14, wie beispielsweise einen Monitor. Das Ultraschall-Bilderzeugungssystem 1 ist insbesondere angepasst, das von der Bilderzeugungseinheit 7 erzeugte Bild anzuzeigen. Zudem können auch andere Bilder auf der Ausgabeeinheit 14 gezeigt werden, wie beispielsweise das oben genannte zuvor aufgenommene transformierte MR-Bild. Auch das zuvor aufgenommene MR-Bild mit dem Indikator, der den Ort des interventionellen Instrumentes anzeigt, kann auf der Ausgabeeinheit 14 gezeigt werden.

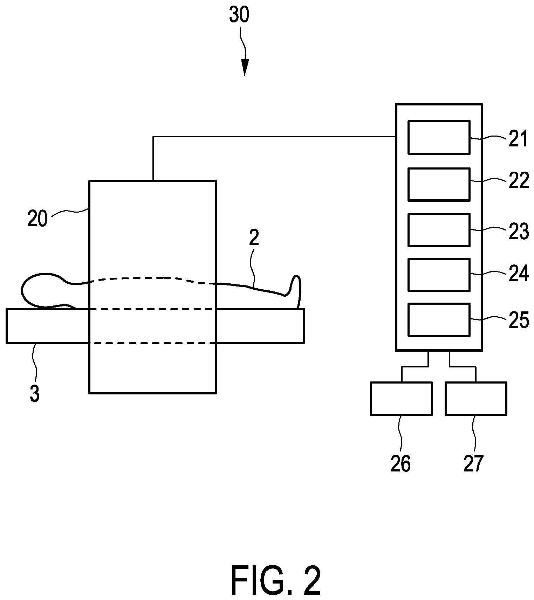

Eine Ausführungsform eines Trainingssystems zum Trainieren eines neuronalen Netzes ist beispielhaft und schematisch in Fig. 2 gezeigt.

Das Trainingssystem 30 umfasst eine Rohdatenaufnahmeeinheit 20 zum Aufnehmen von MR-Rohdaten, Ultraschall-Echo-Rohdaten und Ultraschall-Transmissions-Rohdaten. Zudem umfasst das Trainingssystem 30 eine MR-Steuerungs-und-Rekonstruktionseinheit 21 zum Steuern der Aufnahme der MR-Rohdaten und zum Rekonstruieren eines MR-Bildes auf Basis der MR-Rohdaten. Zudem umfasst das Trainingssystem 30 eine Ultraschall-Steuerungseinheit 22 zum Steuern der Aufnahme der Ultraschall-Echo-Rohdaten und der Ultraschall-Transmissions-Rohdaten und eine Ultraschall-Rekonstruktionseinheit 23 zum Rekonstruieren eines Ultraschalltomographiebildes auf Basis der Ultraschall-Transmissions-Rohdaten und der Ultraschall-Echo-Rohdaten, wobei die Ultraschall-Transmissions-Rohdaten zum Bestimmen der Schallgeschwindigkeiten innerhalb des Patienten verwendet werden und wobei diese Schallgeschwindigkeiten dann bei der Rekonstruktion des Ultraschalltomographiebildes mit Hilfe der Ultraschall-Echo-Rohdaten berücksichtigt werden. Die Ultraschall-Rekonstruktionseinheit 23 ist bevorzugt ausgebildet, das Ultraschalltomographiebild ohne Verwendung eines neuronalen Netzes zu rekonstruieren. Sie verwendet stattdessen bevorzugt bekannte Ultraschalltomographie-Rekonstruktionsalgorithmen. Sie kann aber auch angepasst sein, ein neuronales Netz zur Rekonstruktion zu verwenden. Auch die MR-Steuerungs-und-Rekonstruktionseinheit 21 verwendet für die Rekonstruktion des MR-Bildes auf Basis der MR-Rohdaten bevorzugt kein neuronales Netz, sondern bekannte MR-Rekonstruktionsalgorithmen, die beispielsweise auf eine Fourier-Transformation von einem k-Raum in einen Ortsraum basieren. Auch sie kann aber auch angepasst sein, ein neuronales Netz zur Rekonstruktion zu verwenden.

Da die Rohdatenaufnahmeeinheit 20 angepasst ist, Ultraschall-Echo-Rohdaten und Ultraschall-Transmissions-Rohdaten aufzunehmen, wobei diese Aufnahme der Rohdaten von der Ultraschall-Steuerungseinheit 22 gesteuert wird, können die Rohdatenaufnahmeeinheit 20 und die Ultraschall-Steuerungseinheit 22 als Komponenten einer Ultraschalldatenbereitstellungseinheit zum Bereitstellen von Ultraschallrohdaten des Patienten 2 aufgefasst werden. Da die Rohdatenaufnahmeeinheit 20 zudem MR-Rohdaten aufnimmt und die MR-Steuerungs-und-Rekonstruktionseinheit 21 auf Basis dieser MR-Rohdaten ein MR-Bild des Patienten 2 rekonstruiert, können die Rohdatenaufnahmeeinheit 20 und die MR-Steuerungs-und-Rekonstruktionseinheit 21 als Komponenten einer Bild-Bereitstellungseinheit zum Bereitstellen eines Bildes des Patienten 2 aufgefasst werden, das nicht der Ultraschallbildgebungsmodalität entspricht. Die Rohdatenaufnahmeeinheit 20, die MR-Steuerungs-und-Rekonstruktionseinheit 21 und die Ultraschall-Steuerungseinheit 22 sind so angepasst, dass die MR-Rohdaten, die Ultraschall-Echo-Rohdaten und die Ultraschall-Transmissions-Rohdaten von einem selben Bereich des Patienten 2 gleichzeitig aufgenommen werden.

Das Trainingssystem 30 umfasst des Weiteren eine Zu-Trainierende-Einheit-Bereitstellungseinheit 24 zum Bereitstellen eines zu trainierenden neuronalen Netzes und eine Trainingseinheit 25 zum Trainieren des bereitgestellten neuronalen Netzes derart, dass das trainierte neuronale Netz bei Eingabe der gemessenen Ultraschall-Echo-Rohdaten des Patienten 2 das rekonstruierte MR-Bild ausgibt. Da bei den hier beschriebenen Beispielen die zu trainierenden Einheit ein neuronales Netz ist, kann die Zu-Trainierende-Einheit-Bereitstellungseinheit 24 auch als Neuronales-Netz-Bereitstellungseinheit aufgefasst werden. Die Trainingseinheit 25 ist dabei bevorzugt angepasst, dieses Training des neuronalen Netzes zweistufig durchzuführen. In einer ersten Stufe wird das neuronale Netz derart trainiert, dass das trainierte neuronale Netz bei Eingabe der Ultraschall-Transmissions-Rohdaten des Patienten 2 das rekonstruierte Ultraschalltomographiebild ausgibt. Nach diesem Training wird in einer zweiten Stufe das neuronale Netz derart trainiert, dass das neuronale Netz bei Eingabe der gemessenen Ultraschall-Echo-Rohdaten des Patienten 2 das von der MR-Steuerungs-und-Rekonstruktionseinheit 21 rekonstruierte MR-Bild ausgibt.

Auch hier ist zu beachten, dass die Trainingseinheit 25 das bereitgestellte neuronale Netz zwar mit dem Ziel trainiert, dass bei Eingabe der gemessenen Ultraschall-Echo-Rohdaten des Patienten 2 das rekonstruierte MR-Bild ausgegeben wird, dass dies aber nicht exakt erreicht wird, da das mittels des neuronalen Netzes erzeugte Bild ein Ultraschallbild ist und kein MR-Bild. Das Trainieren mit dem Ziel, dass das neuronale Netz das rekonstruierte MR-Bild ausgibt, führt aber dazu, dass das mittels des trainierten neuronalen Netzes erzeugte Ultraschallbild möglichst gut mit dem rekonstruierten MR-Bild übereinstimmt und insbesondere MR-Bildcharakteristika des Objekts in dem mittel des neuronalen Netzes erzeugten Ultraschallbildes sichtbar sind. Die Trainingseinheit 25 trainiert das bereitgestellte neuronale Netz derart, dass Abweichungen zwischen dem mittels des trainierten neuronalen Netzes erzeugten Ultraschallbild und dem rekonstruierten MR-Bild minimiert werden. Es wird also versucht, Unterschiede zwischen dem MR-Bild und dem mittels des trainierten neuronalen Netzes erzeugten Ultraschallbilds möglichst gering zu machen.

Das Training des neuronalen Netzes wird mir sehr vielen Trainingssätzen durchgeführt, wobei jeder Trainingssatz Ultraschallrohdaten und ein MR-Bild eines Patienten aufweist.

Das Trainingssystem 30 umfasst des Weiteren eine Eingabeeinheit 26 wie eine Tastatur, eine Computermaus, ein berührungssensitives Display, et cetera und eine Ausgabeeinheit 27 wie beispielsweise einen Monitor.

Die Rohdatenaufnahmeeinheit 20 umfasst bspw. Halterungen für Ultraschallsonden zum Aufnehmen der Ultraschall-Echo-Rohdaten und Ultraschall-Transmissions-Rohdaten und diese Ultraschallsonden. Zudem umfasst die Rohdatenaufnahmeeinheit 20 ein MR-System, an dem die Halterungen mit den Ultrasonden befestigt sind. Die Halterungen können auch an dem Patiententisch 3 befestigt sein. Mit flüssigkeitsgefüllten Markern an den Ultraschallsonden kann die Position und Orientierung des jeweiligen Schallkopfes in MR-Bildern gemessen werden. Damit hat man die beiden Koordinatensysteme des MR-System und der Ultraschallsonden registriert. Alternativ können auch andere Trackingsysteme verwendet werden, die auf andere Trackingtechniken basieren, wie bspw. optische Trackingsysteme.

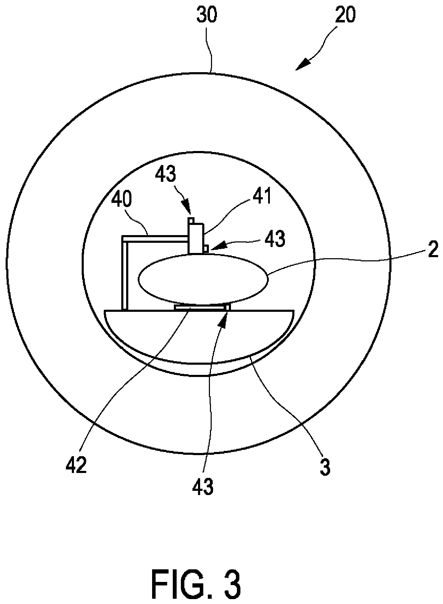

Die Rohdatenaufnahmeeinheit 20 ist etwas detaillierter in Fig. 3 illustriert. Die Rohdatenaufnahmeeinheit 20 umfasst eine MR-Rohdatenaufnahmeeinheit 30, Ultraschallsender und -empfänger 41 und einen Ultraschallempfänger 42 zum Aufnehmen der Ultraschallrohdaten. Zum Aufnehmen der Ultraschall-Transmissions-Rohdaten wird der Ultraschallsender und -empfänger 41 als Ultraschallsender betrieben, wobei die ausgesendeten Ultraschallwellen von dem Ultraschallempfänger 42 empfangen werden. Der Ultraschallsender und -empfänger 41 und der Ultraschallempfänger 42 sind so angeordnet, dass die ausgesendeten Ultraschallwellen den Patienten 2 durchlaufen, bevor sie von dem Ultraschallempfänger 42 empfangen werden. In dieser Ausführungsform ist der Ultraschallsender und -empfänger 41 mittels einer Halterung 40 oberhalb des Patienten 2 angeordnet und der Ultraschallempfänger 42 ist in den Patiententisch 3 integriert. Bevorzugt ist der Ultraschallempfänger 42 so in den Patiententisch 3 integriert, dass der Ultraschallempfänger 42 keine Hervorhebung auf der oberen Fläche des Patiententisches 3 verursacht. Insbesondere ist der Ultraschallempfänger 42 bevorzugt in den Patiententisch 3 eingelassen und beispielsweise hinter einer Verdeckung für den Patienten unsichtbar angeordnet.

MR-Marker 43 können an dem Ultraschallsender und -empfänger 41 und an dem Ultraschallempfänger 42 angebracht sein, um die Position und Orientierung der Ultraschallsender und -empfänger 41, 42 in einem durch die MR-Rohdatenaufnahmeeinheit 30 definierten Koordinatensystem festzustellen. Dies kann sicherstellen, dass sich die Ultraschall-Echo-Rohdaten, die Ultraschall-Transmissions-Rohdaten und die MR-Rohdaten alle auf denselben Bereich des Patienten 2 beziehen. Die entsprechende Registrierung kann beispielsweise von der Ultraschall-Steuerungseinheit 22 oder der MR-Steuerungs-und-Rekonstruktionseinheit 21 durchgeführt werden. Die MR-Marker 43 sind beispielsweise mit einer Flüssigkeit wie Wasser gefüllt, um diese in einem MR-Bild sichtbar zu machen.

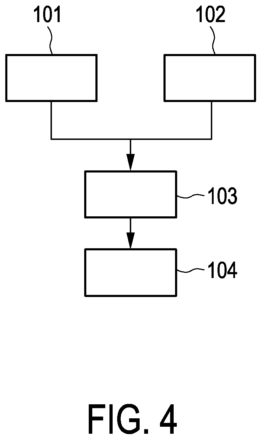

Im Folgenden wird beispielhaft eine Ausführungsform eines Ultraschall-Bilderzeugungsverfahrens zum Erzeugen eines Bildes eines Objekts unter Bezugnahme auf ein Flussdiagramm beschrieben, das in Fig. 4 gezeigt ist.

In Schritt 101 werden Ultraschallrohdaten des Patienten 2 mittels der Ultraschalldatenbereitstellungseinheit 4, 5 bereitgestellt. Insbesondere werden Ultraschall-Echo-Rohdaten mit dem Ultraschallmessgerät 4, das mittels der Steuerungseinheit 5 gesteuert wird, gemessen.

In Schritt 102 wird das neuronale Netz bereitgestellt, das so trainiert worden ist, dass auf Basis von Ultraschall-Echo-Rohdaten ein Ultraschall-Bild erzeugt wird, das nicht der Ultraschallbildgebungsmodalität entspricht. Insbesondere ist das neuronale Netz so trainiert worden, dass auf Basis von Ultraschall-Echo-Rohdaten ein Ultraschallbild mit MR-Bildcharakteristika erzeugt wird.

In Schritt 103 wird ein Bild des Patienten 2 unter Verwendung des in Schritt 102 bereitgestellten neuronalen Netzes auf Basis der in Schritt 101 bereitgestellten Ultraschall-Echo-Rohdaten mittels der Bilderzeugungseinheit 7 erzeugt. Das heißt insbesondere, dass auf Basis der von dem Ultraschallmessgerät 4 gemessenen Ultraschall-Echo-Rohdaten unter Verwendung des neuronalen Netzes ein Ultraschall-Bild mit MR-Charakteristika des Patienten erzeugt wird. In Schritt 104 wird das so erzeugte Bild auf der Ausgabeeinheit 14 angezeigt.

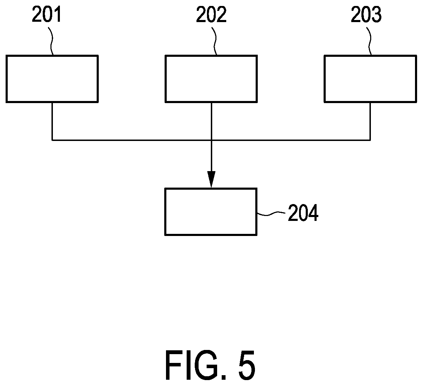

Im Folgenden wird eine Ausführungsform eines Trainingsverfahrens zum Trainieren eines neuronalen Netzes beispielhaft anhand eines Flussdiagramms beschrieben, das in Fig. 5 gezeigt ist.

In Schritt 201 werden Ultraschallrohdaten des Patienten 2 mittels der Ultraschalldatenbereitstellungseinheit 20, 22 bereitgestellt. Insbesondere werden Ultraschall-Echo-Rohdaten und Ultraschall-Transmissions-Rohdaten gleichzeitig von der Rohdatenaufnahmeeinheit 20, die von der Ultraschall-Steuerungseinheit 22 gesteuert wird, aufgenommen. In Schritt 202 wird ein Bild des Patienten 2 mittels der Bild-Bereitstellungseinheit 20, 21 bereitgestellt. Das heißt insbesondere, dass MR-Rohdaten von der Rohdatenaufnahmeeinheit 20 gleichzeitig mit der Messung der Ultraschall-Echo-Rohdaten und Ultraschall-Transmissions-Rohdaten gemessen werden. Die MR-Rohdaten werden dann zu einem MR-Bild rekonstruiert.

In Schritt 203 wird von der Trainierte-Einheit-Bereitstellungseinheit 24 ein neuronales Netz bereitgestellt und in Schritt 204 trainiert die Trainingseinheit 25 das bereitgestellte neuronale Netz derart, dass das trainierte neuronale Netz bei Eingabe der bereitgestellten Ultraschallrohdaten des Patienten 2 das bereitgestellte Bild ausgibt. Das heißt, die Trainingseinheit 25 trainiert das bereitgestellte neuronale Netz mit dem Ziel, dass das trainierte neuronale Netz bei Eingabe der bereitgestellten Ultraschallrohdaten des Patienten 2 das bereitgestellte Bild ausgibt. Da das von dem trainierten neuronalen Netz ausgegebene Bild aber ein Ultraschallbild ist, wird es, wie oben ausgeführt, nicht eins-zu-eins dem bereitgestellten Bild gleichen. Nichtsdestotrotz trainiert die Trainingseinheit 25 das bereitgestellte neuronale Netz mit dem beschriebenen Ziel, indem es versucht, Abweichungen des mittels des trainierten neuronalen Netzes erzeugten Ultraschallbildes von dem bereitgestellten Bild zu minimieren. Dieses Training kann zweistufig durchgeführt werden, wobei die Ultraschall-Transmissions-Rohdaten und die Ultraschall-Echo-Rohdaten dazu verwendet werden können, ein Ultraschalltomographiebild zu rekonstruieren, ohne das neuronale Netz zu verwenden, wonach dann in einer ersten Stufe das neuronale Netz so trainiert wird, dass bei Eingabe der Ultraschall-Echo-Rohdaten das trainierte neuronale Netz das rekonstruierte Ultraschalltomographiebild ausgibt. In einer zweiten Stufe wird das Netz weiter derart trainiert, dass bei Eingabe der Ultraschall-Echo-Rohdaten das rekonstruierte MR-Bild ausgegeben wird.

Obwohl in oben beschriebenen Ausführungsformen das mittels des neuronalen Netzes erzeugte Bild MR-Bildcharakteristika aufweist, kann das neuronale Netz auch trainiert sein, auf Basis der Ultraschallrohdaten Ultraschall-Bilder mit Bildcharakteristika einer anderen Bildgebungsmodalität zu erzeugen wie beispielsweise ein Ultraschall-Bild mit CT-Bildcharakteristika, ein Ultraschall-Bild mit PET-Bildcharakteristika, ein Ultraschall-Bild mit SPECT-Bildcharakteristika oder ein Ultraschall-Bild mit MPI-Bildcharakteristika.

Wenn das neuronale Netz ausgebildet ist, auf Basis der Ultraschallrohdaten ein Ultraschall-Bild mit MR-Bildcharakteristika zu erzeugen, kann das Ultraschall-Bild Bildcharakteristika eines bestimmten MR-Kontrasts aufweisen. Das heißt, das neuronale Netz kann so trainiert sein, dass es auf Basis der Ultraschallrohdaten ein Ultraschall-Bild mit MR-Bildcharakteristika erzeugt, das einen gewünschten Kontrast, das heißt insbesondere einen gewünschten gewebstypischen Kontrast, aufweist. Das Bild kann beispielsweise T1-Bildcharakteristika, T2-Bildcharakteristika, T2*-Bildcharakteristika, Protonen-Dichte-Bildcharakteristika, Perfusions-Bildcharakteristika, Fettgehalt-Bildcharakteristika, Diffusions-Bildcharakteristika, Fluss-Bildcharakteristika, Bewegungs-Bildcharakteristika, Magnetisierungstransfer-Bildcharakteristika, Chemischer-Austausch-Sättigungs-Transfer-Bildcharakteristika, et cetera aufweisen.

Das Ultraschall-Bilderzeugungssystem kann auch angepasst sein, mehrere neuronale Netze bereitzustellen, die jeweils zum Erzeugen eines Ultraschall-Bildes mit Bildcharakteristika einer anderen Bildgebungsmodalität und/oder eines anderen Kontrasttyps einer selben Bildgebungsmodalität trainiert sind. Die Ultraschall-Bildgebungserzeugungseinheit kann auch eine Auswahleinheit aufweisen, die es einem Benutzer ermöglicht, auszuwählen, welcher Bildgebungsmodalität das zu erzeugende Ultraschall-Bild entsprechen soll. Hierdurch hat der Benutzer die Möglichkeit, ein entsprechendes neuronales Netz auszuwählen, das dann zur Bilderzeugung auf Basis der Ultraschallrohdaten verwendet wird.

Nachdem das neuronale Netz trainiert worden ist, ermöglicht es eine sehr schnelle Erzeugung eines Ultraschall-Bildes wie beispielsweise eines Ultraschall-Bildes mit CT-Bildcharakteristika oder mit MR-Bildcharakteristika allein auf Basis der Ultraschallrohdaten. Da zudem die Ultraschallrohdaten sehr schnell aufgenommen werden können, das heißt beispielsweise mit 50 Bildern pro Sekunde, kann das Ultraschall-Bilderzeugungssystem verwendet werden, Ultraschall-Bilder mit CT-Bildcharakteristika, MR-Bildcharakteristika oder Ultraschall-Bilder, die einer anderen Bildgebungsmodalität entsprechen, die nicht die Ultraschallbildgebungsmodalität ist, die zur Aufnahme der Ultraschallrohdaten verwendet worden ist, über einen gewissen Zeitraum mit einer sehr hohen Zeitauflösung aufzunehmen, um beispielsweise eine Bewegung sehr genau zu beobachten. Es wird somit ermöglicht, in Echtzeit Ultraschall-Bilder mit zum Beispiel CT-Bildcharakteristika oder MR-Bildcharakteristika bereitzustellen.

Obwohl in oben beschriebenen Ausführungsformen zum Trainieren des neuronalen Netzes Messdaten verwendet werden, können zum Trainieren auch simulierte Daten, insbesondere simulierte Ultraschallrohdaten und/oder auf einer Simulation basierende Bilder, zum Trainieren verwendet werden. Diese simulierten Daten sind idealisierte Daten, die beispielsweise Bildartefakte nicht aufweisen, die reale Bilder aufweisen könnten. Durch die Verwendung dieser simulierten Daten kann die Qualität des Trainings des neuronalen Netzes weiter verbessert werden, was schließlich zu einer weiter verbesserten Erzeugung beispielsweise eines Ultraschall-Bildes mit CT-Bildcharakteristika oder eines Ultraschall-Bildes mit MR-Bildcharakteristika unter Verwendung des trainierten neuronalen Netzes auf Basis der Ultraschallrohdaten führen kann. Für das Training kann auch eine Kombination aus gemessenen Daten und simulierten Daten verwendet werden.

Obwohl in oben aufgeführten Ausführungsformen das Training zweistufig durchgeführt worden ist, kann das Training auch beispielsweise einstufig durchgeführt werden, wobei das neuronale Netz dann so trainiert wird, dass das neuronale Netz bei Eingabe der Ultraschallrohdaten des Patienten das bereitgestellte Bild einer anderen Bildgebungsmodalität, das heißt beispielsweise ein MR-Bild, das von der MR-Steuerungs-und-Rekonstruktionseinheit 21 rekonstruiert worden ist, ausgibt. Die in oben aufgeführten Ausführungsformen beschriebene erste Stufe des zweistufigen Trainings kann demnach entfallen.

Obwohl in den oben aufgeführten Ausführungsformen auf eine medizinische Bildgebung Bezug genommen worden ist, können die auf Ultraschall basierende Bilderzeugung und das Training auch in anderen Bereichen verwendet werden, wie beispielsweise der Materialforschung oder der Ultraschall-Mikroskopie. Der Patient kann ein menschlicher oder ein tierischer Patient sein. Zudem können das auf Ultraschall basierende Bilderzeugungssystem und das Trainingssystem auch für technische Objekte angewendet werden.

Obwohl in obigen Ausführungsformen die zu trainierende Einheit und die trainierte Einheit neuronale Netze sind, können diese Einheiten auch andere Einheiten sind, die mittels Maschinenlernens (engl. "machine learning") trainiert werden bzw. trainiert worden sind. Diese Einheiten können beispielsweise Hidden Markov Modelle, Systeme zu Supervised Dictionary Learning, Hebbian Learning oder Gradient Descent Learning sein.

In den Ansprüchen schließen die Wörter "aufweisen" und "umfassen" nicht andere Elemente oder Schritte aus und der unbestimmte Artikel "ein" schließt eine Mehrzahl nicht aus.

Ein einzelnes System, eine einzelne Einheit oder eine einzelne Vorrichtung kann die Funktionen mehrerer Elemente durchführen, die in den Ansprüchen aufgeführt sind. Die Tatsache, dass einzelne Funktionen und Elemente in unterschiedlichen abhängigen Ansprüchen aufgeführt sind, bedeutet nicht, dass nicht auch eine Kombination dieser Funktionen oder Elemente vorteilhaft verwendet werden könnte.

Vorgänge wie das Bereitstellen von Ultraschallrohdaten, das Bereitstellen des neuronalen Netzes, das Erzeugen eines Bildes, et cetera, die von einer oder mehreren Einheiten oder Vorrichtungen durchgeführt werden, können auch von einer anderen Anzahl an Einheiten oder Vorrichtungen durchgeführt werden. Diese Vorgänge und/oder die Steuerung des Ultraschall-Bilderzeugungssystems gemäß dem Ultraschall-Bilderzeugungsverfahren und/oder die Steuerung des Trainingssystems gemäß dem Trainingsverfahren können als Programmcode eines Computerprogramms und/oder als entsprechende Hardware implementiert sein.

Ein Computerprogramm kann auf einem geeigneten Medium gespeichert und/oder verteilt werden, wie beispielsweise einem optischen Speichermedium oder einem Festkörperspeichermedium, das zusammen mit oder als Teil anderer Hardware vertrieben wird. Das Computerprogramm kann aber auch in anderen Formen vertrieben werden, beispielsweise über das Internet oder andere Telekommunikationssysteme.

Die Bezugszeichen in den Ansprüchen sind nicht derart zu verstehen, dass der Gegenstand und der Schutzbereich der Ansprüche durch diese Bezugszeichen eingeschränkt sind.

## Patentansprüche

1. Ultraschall-Bilderzeugungssystem zum Erzeugen eines Bildes eines Objekts, wobei das Ultraschall-Bilderzeugungssystem (1) aufweist:
- eine Ultraschalldatenbereitstellungseinheit (4, 5) zum Bereitstellen von Ultraschallrohdaten des Objekts (2), die mit einer Ultraschallbildungsmodalität aufgenommen worden sind,
- eine Trainierte-Einheit-Bereitstellungseinheit (6) zum Bereitstellen einer mittels Maschinenlernens trainierten Einheit, die angepasst ist, auf Basis von Ultraschallrohdaten eines Objekts ein Ultraschall-Bild des Objekts zu erzeugen, das nicht der Ultraschallbildgebungsmodalität entspricht, die zur Aufnahme der Ultraschallrohdaten verwendet worden ist, und
- eine Bilderzeugungseinheit (7) zum Erzeugen des Bildes des Objekts (2) unter Verwendung der bereitgestellten trainierten Einheit auf Basis der bereitgestellten Ultraschallrohdaten des Objekts (2).

2. Ultraschall-Bilderzeugungssystem nach Anspruch 1, **dadurch gekennzeichnet, dass** die Ultraschalldatenbereitstellungseinheit (4, 5) angepasst ist, als Ultraschallrohdaten Ultraschall-Echo-Rohdaten bereitzustellen, die mit einer Ultraschall-Echo-Bildungsmodalität aufgenommen worden sind, wobei die Trainierte-Einheit-Bereitstellungseinheit (6) angepasst ist, als die trainierte Einheit eine trainierte Einheit bereitzustellen, die angepasst ist, auf Basis von Ultraschallrohdaten eines Objekts ein Bild des Objekts mit Bildcharakteristika zu erzeugen, die ausgewählt sind aus einer Gruppe bestehend aus Computertomographie-Bildcharakteristika, Magnetresonanz-Bildcharakteristika, Positronen-Emissions-Tomographie-Bildcharakteristika, Einzelphotonen-Emissions-Computertomographie-Bildcharakteristika und Magnetpartikel-Bildcharakteristika, und wobei die Trainierte-Einheit-Bereitstellungseinheit (6) angepasst ist, als die mittels Maschinenlernens trainierte Einheit ein neuronales Netz bereitzustellen, das angepasst ist, auf Basis von Ultraschallrohdaten eines Objekts ein Ultraschall-Bild des Objekts zu erzeugen, das nicht der Ultraschallbildgebungsmodalität entspricht, das zur Aufnahme der Ultraschallrohdaten verwendet worden ist.

3. Ultraschall-Bilderzeugungssystem nach Anspruch 2, **dadurch gekennzeichnet, dass** die Trainierte-Einheit-Bereitstellungseinheit (6) angepasst ist, als das neuronale Netz ein nicht vollständig faltendes neuronales Netz bereitzustellen.

4. Ultraschall-Bilderzeugungssystem nach einem der vorhergehenden Ansprüche, **dadurch gekennzeichnet, dass** das Ultraschall-Bilderzeugungssystem (1) eine Bild-Bereitstellungseinheit (8) zum Bereitstellen eines weiteren Bildes des Objekts (2) aufweist, das nicht der Ultraschallbildgebungsmodalität entspricht, wobei dieses weitere Bild nicht unter Verwendung der trainierten Einheit erzeugt worden ist, und wobei das Ultraschall-Bilderzeugungssystem (1) eine Registrierungseinheit (9) zum Registrieren des bereitgestellten weiteren Bildes und des unter Verwendung der trainierten Einheit erzeugten Bildes miteinander aufweist.

5. Ultraschall-Bilderzeugungssystem nach Anspruch 4, **dadurch gekennzeichnet, dass** das Ultraschall-Bilderzeugungssystem (1) aufweist:
- eine Elementbestimmungseinheit (10) zum Bestimmen eines Ortes eines Elements in dem unter Verwendung der trainierten Einheit erzeugten Bild und
- eine Indikatorerzeugungseinheit (11) zum Erzeugen eines Indikators, der den Ort des Elements in dem weiteren bereitgestellten Bild anzeigt, auf Basis der Registrierung und des bestimmten Ortes.

6. Trainingssystem zum Trainieren einer Einheit mittels Maschinenlernens, wobei das Trainingssystem (30) aufweist:
- eine Ultraschalldatenbereitstellungseinheit (20, 22) zum Bereitstellen von Ultraschallrohdaten eines Objekts (2), die mit einer Ultraschallbildungsmodalität aufgenommen worden sind oder die durch Simulation einer Aufnahme mit der Ultraschallbildungsmodalität erzeugt worden sind,
- eine Zu-Trainierende-Einheit-Bereitstellungseinheit (24) zum Bereitstellen einer mittels Maschinenlernens zu trainierende Einheit,
- eine Bild-Bereitstellungseinheit (20, 21) zum Bereitstellen eines Bildes des Objekts (2), das nicht mit der Ultraschallbildungsmodalität aufgenommen worden ist, mit der die Ultraschallrohdaten aufgenommen worden sind, und nicht durch Simulation einer Aufnahme mit der Ultraschallbildungsmodalität erzeugt worden ist,
- eine Trainingseinheit (25) zum Trainieren der bereitgestellten zu trainierenden Einheit derart, dass die trainierte Einheit bei Eingabe der bereitgestellten Ultraschallrohdaten des Objekts (2) das bereitgestellte Bild ausgibt.

7. Trainingssystem nach Anspruch 6, **dadurch gekennzeichnet, dass** die Ultraschalldatenbereitstellungseinheit (20, 22) und die Bild-Bereitstellungseinheit (20, 21) angepasst sind, die Ultraschallrohdaten und das Bild von einem selben Bereich des Objekts (2), insbesondere gleichzeitig, aufzunehmen.

8. Trainingssystem nach einem der Ansprüche 6 und 7, **dadurch gekennzeichnet, dass** die Trainingseinheit (25) angepasst ist, a) zunächst auf Basis der bereitgestellten Ultraschallrohdaten ein Ultraschallbild des Objekts (2) zu erzeugen, b) dann die zu trainierende Einheit derart zu trainieren, dass die trainierte Einheit bei Eingabe der bereitgestellten Ultraschallrohdaten des Objekts (2) das erzeugte Ultraschall-Bild ausgibt, und c) schließlich die Einheit derart zu trainieren, dass die trainierte Einheit bei Eingabe der bereitgestellten Ultraschallrohdaten des Objekts (2) das bereitgestellte Bild ausgibt, das von der Bild-Bereitstellungseinheit bereitgestellt worden ist.

9. Trainingssystem nach einem der Ansprüche 6 bis 8 **dadurch gekennzeichnet, dass** die Ultraschalldatenbereitstellungseinheit (20, 22) angepasst ist, Ultraschall-Echo-Rohdaten und Ultraschall-Transmissions-Rohdaten als Ultraschallrohdaten bereitzustellen.

10. Trainingssystem nach den Ansprüchen 8 und 9, **dadurch gekennzeichnet, dass** die Trainingseinheit (25) angepasst ist, a) zunächst auf Basis der bereitgestellten Ultraschall-Transmissions-Rohdaten ein Ultraschalltomographiebild des Objekts (2) zu erzeugen, b) dann die Einheit derart zu trainieren, dass die trainierte Einheit bei Eingabe der bereitgestellten Ultraschall-Transmissions-Rohdaten des Objekts (2) das erzeugte Ultraschalltomographiebild ausgibt, und c) schließlich die Einheit derart zu trainieren, dass die trainierte Einheit bei Eingabe der bereitgestellten Ultraschall-Echo-Rohdaten des Objekts (2) das bereitgestellte Bild ausgibt, das von der Bild-Bereitstellungseinheit bereitgestellt worden ist.

11. Eine durch ein Trainingssystem gemäß einem der Ansprüche 6 bis 10 mittels Maschinenlernens trainierte Einheit, die angepasst ist, auf Basis von Ultraschallrohdaten eines Objekts ein Ultraschall-Bild des Objekts zu erzeugen, das nicht der Ultraschallbildgebungsmodalität entspricht, das zur Aufnahme der Ultraschallrohdaten verwendet worden ist.

12. Ultraschall-Bilderzeugungsverfahren zum Erzeugen eines Bildes eines Objekts, wobei das Bilderzeugungsverfahren aufweist:
- Bereitstellen von Ultraschallrohdaten des Objekts (2), die mit einer Ultraschallbildungsmodalität aufgenommen worden sind, mittels einer Ultraschalldatenbereitstellungseinheit (4, 5),
- Bereitstellen einer mittels Maschinenlernens trainierten Einheit, die angepasst ist, auf Basis von Ultraschallrohdaten eines Objekts ein Ultraschall-Bild des Objekts zu erzeugen, das nicht der Ultraschallbildgebungsmodalität entspricht, mit der die Ultraschallrohdaten aufgenommen worden sind, mittels einer Trainierte-Einheit-Bereitstellungseinheit (6) und
- Erzeugen des Bildes des Objekts (2) unter Verwendung der bereitgestellten trainierten Einheit auf Basis der bereitgestellten Ultraschallrohdaten des Objekts (2) mittels einer Bilderzeugungseinheit (7).

13. Trainingsverfahren zum Trainieren einer Einheit mittels Maschinenlernens, wobei das Trainingsverfahren aufweist:
- Bereitstellen von Ultraschallrohdaten eines Objekts (2) mittels einer Ultraschalldatenbereitstellungseinheit (20, 22), die mit einer Ultraschallbildungsmodalität aufgenommen worden sind oder die durch Simulation einer Aufnahme mit der Ultraschallbildungsmodalität erzeugt worden sind,
- Bereitstellen einer zu trainierenden Einheit mittels einer Trainierte-Einheit-Bereitstellungseinheit (24),
- Bereitstellen eines Bildes des Objekts (2), das nicht mit der Ultraschallbildungsmodalität aufgenommen worden ist, mit der die Ultraschallrohdaten aufgenommen worden sind, und nicht durch Simulation einer Aufnahme mit der Ultraschallbildungsmodalität erzeugt worden ist, mittels einer Bild-Bereitstellungseinheit (20, 21),
- Trainieren der bereitgestellten Einheit derart, dass die trainierte Einheit bei Eingabe der bereitgestellten Ultraschallrohdaten des Objekts (2) das bereitgestellte Bild ausgibt, mittels einer Trainingseinheit (25).

14. Computerprogramm zum Erzeugen eines Bildes eines Objekts, wobei das Computerprogramm angepasst ist, das Ultraschall-Bilderzeugungsverfahren gemäß Anspruch 12 durchzuführen, wenn es auf einem Ultraschall-Bilderzeugungssystem zum Erzeugen von Bildern gemäß einem der Ansprüche 1 bis 5 ausgeführt wird.

15. Computerprogramm zum Trainieren einer Einheit mittels Maschinenlernens, wobei das Computerprogramm angepasst ist, das Trainingsverfahren zum Trainieren einer Einheit mittels Maschinenlernens gemäß Anspruch 13 durchzuführen, wenn es auf einem Trainingssystem (30) zum Trainieren einer Einheit mittels Maschinenlernens gemäß einem der Ansprüche 6 bis 10 ausgeführt wird.

## Claims

1. An ultrasound image generating system for generating an image of an object, wherein the ultrasound image generating system (1) comprises:
- an ultrasound data provisioning unit (4, 5) for providing raw ultrasound data of the object (2), which data have been acquired by means of an ultrasound imaging modality,
- a trained unit provisioning unit (6) for providing a unit trained by machine learning, which is adapted to generate an ultrasound image of an object on the basis of raw ultrasound data of the object, which image does not correspond to the ultrasound imaging modality that was used to acquire the raw ultrasound data, and
- an image generating unit (7) for generating the image of the object (2) using the provided trained unit, on the basis of the provided raw ultrasound data of the object (2).

2. The ultrasound image generating system according to claim 1, **characterized in that** the ultrasound data provisioning unit (4, 5) is adapted to provide raw ultrasound echo data acquired by means of an ultrasound echo imaging modality as the raw ultrasound data, wherein the trained unit provisioning unit (6) is adapted to provide, as the trained unit, a trained unit which is adapted to generate an image of an object on the basis of raw ultrasound data of the object, with image characteristics selected from a group consisting of computed tomography image characteristics, magnetic resonance image characteristics, positron emission tomography image characteristics, single-photon emission computed tomography image characteristics and magnetic particle image characteristics, and wherein the trained unit provisioning unit (6) is adapted to provide, as the unit trained by machine learning, a neural network which is adapted to generate an ultrasound image of an object on the basis of raw ultrasound data of the object, which image does not correspond to the ultrasound imaging modality that was used to acquire the raw ultrasound data.

3. The ultrasound image generating system according to claim 2, **characterized in that** the trained unit provisioning unit (6) is adapted to provide a non-fully convolutional neural network as the neural network.

4. The ultrasound image generating system according to any one of the preceding claims, **characterized in that** the ultrasound image generating system (1) has an image providing unit (8) for providing a further image of the object (2), which image does not correspond to the ultrasound imaging modality, wherein this further image was not generated using the trained unit, and wherein the ultrasound image generating system (1) has a registration unit (9) for registering with each other the further image provided and the image generated using the trained unit.

5. The ultrasound image generating system according to claim 4, **characterized in that** the ultrasound image generating system (1) comprises:
- an element identification unit (10) for identifying a location of an element in the image generated using the trained unit and
- an indicator generating unit (11) for generating an indicator which indicates the location of the element in the further image provided, on the basis of the registration and the identified location.

6. A training system for training a unit by machine learning, wherein the training system (30) comprises:
- an ultrasound data provisioning unit (20, 22) for providing raw ultrasound data of an object (2), which have been acquired by means of an ultrasound imaging modality or which have been generated by simulating acquisition by means of the ultrasound imaging modality,
- a unit-to-be-trained provisioning unit (24) for providing a unit to be trained by machine learning,
- an image providing unit (20, 21) for providing an image of the object (2) which has not been acquired by means of the ultrasound imaging modality with which the raw ultrasound data were acquired, and which has not been generated by simulating acquisition by means of the ultrasound imaging modality,
- a training unit (25) for training the provided unit-to-be-trained, such that the trained unit outputs the provided image when the provided raw ultrasound data of the object (2) are inputted.

7. The training system according to claim 6, **characterized in that** the ultrasound data provisioning unit (20, 22) and the image providing unit (20, 21) are adapted to acquire, in particular simultaneously, the raw ultrasound data and the image of a same area of the object (2).

8. The training system according to any one of claims 6 and 7, **characterized in that** the training unit (25) is adapted a) to firstly generate an ultrasound image of the object (2) on the basis of the raw ultrasound data provided, b) to then train the unit-to-be-trained such that the trained unit outputs the generated ultrasound image when the provided raw ultrasound data of the object (2) are inputted, and finally c) to train the unit such that when the provided raw ultrasound data of the object (2) are inputted, the trained unit outputs the image provided by the image providing unit.

9. The training system according to any one of claims 6 to 8, **characterized in that** the ultrasound data provisioning unit (20, 22) is adapted to provide raw ultrasound echo data and raw ultrasound transmission data as raw ultrasound data.

10. The training system according to claims 8 and 9, **characterized in that** the training unit (25) is adapted, a) to firstly generate an ultrasound tomography image of the object (2) on the basis of the raw ultrasound transmission data provided, b) to then train the unit such that the trained unit outputs the generated ultrasound tomography image when the raw ultrasound transmission data provided are inputted, and finally c) to train the unit such that when the provided raw ultrasound echo data of the object (2) are inputted, the trained unit outputs the image provided by the image providing unit.

11. A unit trained by a training system according to any one of claims 6 to 10 by means of machine learning, which is adapted to generate an ultrasound image of an object on the basis of raw ultrasound data of the object, which image does not correspond to the ultrasound imaging modality that was used to acquire the raw ultrasound data.

12. An ultrasound image generating method for generating an image of an object, wherein the ultrasound image generating method comprises:
- providing raw ultrasound data of the object (2), which have been acquired by means of an ultrasound imaging modality, by means of an ultrasound data provisioning unit (4, 5),
- providing, by means of a trained unit provisioning unit (6), a unit trained by machine learning, which is adapted to generate an ultrasound image of an object on the basis of raw ultrasound data of the object, which image does not correspond to the ultrasound imaging modality with which the raw ultrasound data were acquired, and
- generating the image of the object (2), by means of an image generating unit (7), using the provided trained unit and on the basis of the provided raw ultrasound data of the object (2).

13. A training method for training a unit by machine learning, wherein the training method comprises:
- providing raw ultrasound data of an object (2) by means of an ultrasound data provisioning unit (20, 22), which have been acquired by means of an ultrasound imaging modality or have been generated by simulating acquisition by means of the ultrasound imaging modality,
- providing a unit-to-be-trained by means of a trained unit provisioning unit (24),
- providing an image of the object (2) by means of an image providing unit (20, 21), which image has not been acquired by means of the ultrasound imaging modality with which the raw ultrasound data were acquired, and which has not been generated by simulating acquisition by means of the ultrasound imaging modality,
- training, by means of a training unit (25), the provided unit, such that the trained unit outputs the provided image when the provided raw ultrasound data of the object (2) are inputted.

14. A computer program for generating an image of an object, wherein the computer program is adapted to carry out the ultrasound image generating method according to claim 12 when it is run on an ultrasound image generating system for generating images in accordance with any one of claims 1 to 5.

15. A computer program for training a unit by machine learning, wherein the computer program is adapted to carry out the training method for training a unit by machine learning in accordance with claim 13 when it is run on a training system (30) for training a unit by machine learning in accordance with any one of claims 6 to 10.

## Revendications

1. Système d'imagerie par ultrasons pour la génération d'une image d'un objet, dans lequel le système d'imagerie par ultrasons (1) présente :
- une unité de fourniture de données ultrasonores (4, 5) pour la fourniture de données brutes ultrasonores de l'objet (2) qui ont été enregistrées avec une modalité d'imagerie par ultrasons,
- une unité de fourniture d'unité entraînée (6) pour la fourniture d'une unité entraînée au moyen d'un apprentissage machine qui est adaptée afin de générer, sur la base de données brutes ultrasonores d'un objet, une image ultrasonore de l'objet qui ne correspond pas à la modalité d'imagerie par ultrasons qui a été utilisée pour l'enregistrement des données brutes ultrasonores, et
- une unité d'imagerie (7) pour la génération de l'image de l'objet (2) en utilisant l'unité entraînée fournie sur la base des données brutes ultrasonores fournies de l'objet (2).

2. Système d'imagerie par ultrasons selon la revendication 1, **caractérisé en ce que** l'unité de fourniture de données ultrasonores (4, 5) est adaptée afin de fournir, comme données brutes ultrasonores, des données brutes d'écho par ultrasons qui ont été enregistrées avec une modalité de formation d'écho par ultrasons, dans lequel l'unité de fourniture d'unité entraînée (6) est adaptée afin de fournir, comme l'unité entraînée, une unité entraînée qui est adaptée afin de générer, sur la base de données brutes ultrasonores d'un objet, une image de l'objet avec des caractéristiques d'image qui sont sélectionnées à partir d'un groupe composé de caractéristiques d'image de tomodensitométrie, caractéristiques d'image de résonance magnétique, caractéristiques d'image de tomographie d'émission de positrons, caractéristiques d'image de tomodensitométrie d'émission de photons individuels et caractéristiques d'image de particules magnétiques, et dans lequel l'unité de fourniture d'unité entraînée (6) est adaptée afin de fournir, comme l'unité entraînée au moyen de l'apprentissage machine, un réseau neuronal qui est adapté afin de générer, sur la base de données brutes ultrasonores d'un objet, une image ultrasonore de l'objet qui ne correspond pas à la modalité d'imagerie par ultrasons qui a été utilisée pour l'enregistrement des données brutes ultrasonores.

3. Système d'imagerie par ultrasons selon la revendication 2, **caractérisé en ce que** l'unité de fourniture d'unité entraînée (6) est adaptée afin de fournir, comme réseau neuronal, un réseau neuronal à convolution non complète.

4. Système d'imagerie par ultrasons selon l'une quelconque des revendications précédentes, **caractérisé en ce que** le système d'imagerie par ultrasons (1) présente une unité de fourniture d'image (8) pour la fourniture d'une autre image de l'objet (2) qui ne correspond pas à la modalité d'imagerie par ultrasons, dans lequel cette autre image n'a pas été générée en utilisant l'unité entraînée, et dans lequel le système d'imagerie par ultrasons (1) présente une unité d'enregistrement (9) pour l'enregistrement de l'autre image fournie et de l'image générée en utilisant l'unité entraînée entre elles.

5. Système d'imagerie par ultrasons selon la revendication 4, **caractérisé en ce que** le système d'imagerie par ultrasons (1) présente :
- une unité de détermination d'élément (10) pour la détermination d'un endroit d'un élément dans l'image générée en utilisant l'unité entraînée et
- une unité de génération d'indicateur (11) pour la génération d'un indicateur qui affiche l'endroit de l'élément dans l'autre image fournie, sur la base de l'enregistrement et de l'endroit déterminé.

6. Système d'entraînement pour l'entraînement d'une unité au moyen de l'apprentissage machine, dans lequel le système d'entraînement (30) présente :
- une unité de fourniture de données ultrasonores (20, 22) pour la fourniture de données brutes ultrasonores d'un objet (2) qui ont été enregistrées avec une modalité d'imagerie par ultrasons ou qui ont été générées par la simulation d'un enregistrement avec la modalité d'imagerie par ultrasons,
- une unité de fourniture d'unité à entraîner (24) pour la fourniture d'une unité à entraîner au moyen de l'apprentissage machine,
- une unité de fourniture d'image (20, 21) pour la fourniture d'une image de l'objet (2) qui n'a pas été enregistrée avec la modalité d'imagerie par ultrasons, avec laquelle les données brutes ultrasonores ont été enregistrées, et n'a pas été générée par simulation d'un enregistrement avec la modalité d'imagerie par ultrasons,
- une unité d'entraînement (25) pour l'entraînement de l'unité à entraîner fournie de telle manière que l'unité entraînée sorte l'image fournie lors de la saisie des données brutes ultrasonores fournies de l'objet (2).

7. Système d'entraînement selon la revendication 6, **caractérisé en ce que** l'unité de fourniture de données ultrasonores (20, 22) et l'unité de fourniture d'image (20, 21) sont adaptées afin d'enregistrer les données brutes ultrasonores et l'image d'une même zone de l'objet (2) en particulier simultanément.

8. Système d'entraînement selon l'une quelconque des revendications 6 et 7, **caractérisé en ce que** l'unité d'entraînement (25) est adaptée afin de générer a) tout d'abord, sur la base des données brutes ultrasonores fournies, une image ultrasonore de l'objet (2), b) ensuite d'entraîner l'unité à entraîner de telle manière que l'unité entraînée sorte l'image ultrasonore générée lors de la saisie des données brutes ultrasonores fournies de l'objet (2), et c) d'entraîner finalement l'unité de telle manière que l'unité entraînée sorte, lors de la saisie des données brutes ultrasonores fournies de l'objet (2), l'image fournie qui a été fournie par l'unité de fourniture d'image.

9. Système d'entraînement selon l'une quelconque des revendications 6 à 8, **caractérisé en ce que** l'unité de fourniture de données ultrasonores (20, 22) est adaptée afin de fournir des données brutes d'écho par ultrasons et des données brutes de transmission d'ultrasons comme données brutes ultrasonores.

10. Système d'entraînement selon les revendications 8 et 9, **caractérisé en ce que** l'unité d'entraînement (25) est adaptée afin de générer a) tout d'abord, sur la base des données brutes de transmission d'ultrasons fournies, une image tomographique ultrasonore de l'objet (2), b) ensuite d'entraîner l'unité de telle manière que l'unité entraînée sorte l'image tomographique ultrasonore générée lors de la saisie des données brutes de transmission ultrasonores fournies de l'objet (2), et c) d'entraîner finalement l'unité de telle manière que l'unité entraînée sorte, lors de la saisie des données brutes d'écho par ultrasons fournies de l'objet (2), l'image fournie qui a été fournie par l'unité de fourniture d'image.

11. Unité entraînée par un système d'entraînement selon l'une quelconque des revendications 6 à 10 au moyen de l'apprentissage machine qui est adaptée afin de générer, sur la base de données brutes ultrasonores d'un objet, une image ultrasonore de l'objet qui ne correspond pas à la modalité d'imagerie par ultrasons qui a été utilisée pour l'enregistrement des données brutes ultrasonores.

12. Procédé d'imagerie par ultrasons pour la génération d'une image d'un objet, dans lequel le procédé d'imagerie présente :
- la fourniture de données brutes ultrasonores de l'objet (2) qui ont été enregistrées avec une modalité d'imagerie par ultrasons, au moyen d'une unité de fourniture de données ultrasonores (4, 5),
- la fourniture d'une unité entraînée au moyen de l'apprentissage machine qui est adaptée afin de générer, sur la base de données brutes ultrasonores d'un objet, une image ultrasonore de l'objet qui ne correspond pas à la modalité d'imagerie par ultrasons, avec laquelle les données brutes ultrasonores ont été enregistrées, au moyen d'une unité de fourniture d'unité entraînée (6) et
- la génération de l'image de l'objet (2) en utilisant l'unité entraînée fournie sur la base des données brutes ultrasonores fournies de l'objet (2) au moyen d'une unité d'imagerie (7).

13. Procédé d'entraînement pour l'entraînement d'une unité au moyen de l'apprentissage machine, dans lequel le procédé d'entraînement présente :
- la fourniture de données brutes ultrasonores d'un objet (2) au moyen d'une unité de fourniture de données ultrasonores (20, 22) qui ont été enregistrées avec une modalité d'imagerie par ultrasons ou qui ont été générées par simulation d'un enregistrement avec la modalité d'imagerie par ultrasons,
- la fourniture d'une unité à entraîner au moyen d'une unité de fourniture d'unité entraînée (24),
- la fourniture d'une image de l'objet (2) qui n'a pas été enregistrée avec la modalité d'imagerie par ultrasons, avec laquelle les données brutes ultrasonores ont été enregistrées, et n'a pas été générée par simulation d'un enregistrement avec la modalité d'imagerie par ultrasons, au moyen d'une unité de fourniture d'image (20, 21),
- l'entraînement de l'unité fournie de telle manière que l'unité entraînée sorte, lors de la saisie des données brutes ultrasonores fournies de l'objet (2), l'image fournie, au moyen d'une unité d'entraînement (25).

14. Programme informatique pour la génération d'une image d'un objet, dans lequel le programme informatique est adapté afin de réaliser le procédé de d'imagerie par ultrasons selon la revendication 12, lorsqu'il est réalisé sur un système d'imagerie par ultrasons pour la génération d'images selon l'une quelconque des revendications 1 à 5.

15. Programme informatique pour l'entraînement d'une unité au moyen de l'apprentissage machine, dans lequel le programme informatique est adapté afin de réaliser le procédé d'entraînement pour l'entraînement d'une unité au moyen de l'apprentissage machine selon la revendication 13 lorsqu'il est réalisé sur un système d'entraînement (30) pour l'entraînement d'une unité au moyen de l'apprentissage machine selon l'une quelconque des revendications 6 à 10.
